# EUROPEAN PATENT APPLICATION

(11) **EP 4 810 524 A1**
(43) Date of publication of application: **23.09.2026**
(21) Application number: 24890850.1
(22) Date of filing: 18.11.2024
(51) Int. Cl.: C07K 19/00, C12N 15/62, C12N 15/867, C12N 5/10, A61K 39/00, A61P 35/00, A61P 35/02

(54) **BISPECIFIC CHIMERIC ANTIGEN RECEPTOR TARGETING CD38 AND CLL1, AND USE THEREOF**

(30) Priority: 17.11.2023 CN 202311533985
(71) Applicant: Gracell Bioscience (Shanghai) Co., Ltd., Shanghai 200233 (CN)
(72) Inventor: TANG, Jiaxing, Shanghai 200233 (CN); YIN, Wenjie, Shanghai 200233 (CN); DONG, Qi, Shanghai 200233 (CN); SHEN, Lianjun, Shanghai 200233 (CN); CAO, Wei, Shanghai 200233 (CN)
(74) Representative: AstraZeneca Intellectual Property
(86) International application number: PCT/CN2024/132542
(87) International publication number: WO 2025/103498

(57) **Abstract**

A bispecific chimeric antigen receptor targeting CD38 and CLL1 is provided. The chimeric antigen receptor at least includes, from the N-terminus to the C-terminus: i) an antigen-binding domain that specifically recognizes CLL1 and CD38; ii) a transmembrane domain; iii) at least one co-stimulatory domain; and iv) a signaling domain. A dual-targeting CAR-T cell prepared by using the chimeric antigen receptor described above shows highly specific in vitro cytotoxicity against CLL1-positive and/or CD38-positive target cells.

## Description

### TECHNICAL FIELD

The present invention belongs to the field of immune cell therapy, and specifically relates to a bispecific chimeric antigen receptor targeting CD38 and CLL1, and use thereof.

### BACKGROUND ART

Acute myelogenous leukemia (AML) is a cancer of the myeloid blood cells characterized by the clonal expansion of malignant hematopoietic cells. These malignant hematopoietic cells accumulate in the bone marrow and blood and interfere with normal blood cells. The biological and clinical complexity of AML derives from the multiple subtypes of AML, which greatly increases the difficulty of AML treatment. Common treatment methods for AML include chemotherapy and hematopoietic stem cell transplantation. However, since AML is most prevalent in the elderly, most of the patients cannot receive intensive chemotherapy. On the other hand, stem cell transplantation suffers from a limited source of donors. As a result, the patients have an extremely low survival rate and a high relapse rate. There is a need to seek a new therapeutic approach for the treatment.

A chimeric antigen receptor (CAR) is an artificial receptor that mimics the function of a T cell receptor, and comprises an antigen-recognizing fragment (or an antigen-corresponding ligand) of an antibody fused to a downstream signal domain of a T cell. Under the action of the chimeric receptor, T cells are specifically targeted to a tumor-related or specific antigen expressed on the surface of tumor cells, and activation and expansion of and subsequent tumor killing by CAR-T cells are induced.

CD38 antigen is a type II transmembrane glycoprotein of 45 kDa in size capable of catalyzing the synthesis and degradation of cyclic adenosine diphosphate-ribose. CD38 is also involved in the regulation of cell apoptosis and cell expansion, and has important adhesion properties. Most AML blast cells have a high expression of CD38, making them a potential target for AML therapy.

C-type lectin-like 1 (CLL1) is a glycoprotein receptor, and is a member of the large family of C-type lectin-like receptors involved in immunomodulation. CLL1 is mainly expressed in hematopoietic cells, and mainly in innate immune cells including monocytes, DCs and granulocytes, and bone marrow progenitor cells. CLL1 is also found in acute myeloid leukemia (AML) blast cells, leukemia stem cells (e.g., CD34+/CD38-), and a small fraction of hematopoietic progenitor cells (CD34+/CD38+ or CD34+/CD33+), but is not expressed on normal hematopoietic stem cells (CD34+/CD38- or CD34+/CD33-).

Due to the lack of suitable targeted surface antigens, breakthroughs in the treatment of AML with CAR-T cells have long remained elusive. Since targeted AML antigens are frequently co-expressed on healthy hematopoietic stem/progenitor cells (HSPCs), the result would be the loss of all myeloid progeny cells. Innovative solutions are being sought to overcome these barriers so as to make CART therapy a viable option for AML patients.

### SUMMARY OF THE INVENTION

In order to solve the above-mentioned problems, the present invention provides the following technical solutions:
A first aspect of the present invention discloses a bispecific chimeric antigen receptor targeting CD38 and CLL1, a fusion protein of the chimeric antigen receptor at least comprises, from the N-terminus to the C-terminus:
i) an antigen-binding domain that specifically binds to CD38 and/or CLL1;
ii) a transmembrane domain;
iii) at least one co-stimulatory domain; and
iv) a signaling domain.

Preferably, the above-described antigen-binding domain is monovalent or multivalent; that is, the antigen-binding domain comprises a single antigenic determinant or multiple antigenic determinants, and can specifically bind to a single antigenic epitope or multiple antigenic epitopes.

Preferably, the above-described antigen domain comprises a single-domain antibody; others may also be selected and used, such as camel Ig, IgNAR, Fab fragment, Fab' fragment, F(ab')z fragment, F(ab')3 fragment, Fv, single chain antibody (e.g., scFv, di-scFv, (scFv)z), minibody, difunctional antibody, trifunctional antibody, tetrafunctional antibody, disulfide bond-stabilized Fv protein ("dsFv"), chimeric antibody, humanized antibody, single domain antibody, bispecific antibody or multispecific antibody, binding ligand, or protein domain.

Preferably, the antigen domain comprises two single-domain antibodies, for example, VHH1, which represents a single-domain antibody of a first antigen-binding domain, and VHH2, which represents a single-domain antibody of a second antigen-binding domain; the first antigen-binding domain targets and binds to CD38, and the second antigen-binding domain targets and binds to CLL1.
the first antigen-binding domain and the second antigen-binding domain are arranged, from the amino terminus to the carboxyl terminus, in one of the patterns selected from the following group:
i) VHH1-VHH2; or
ii) VHH2-VHH1.

In the present application, the single-domain antibody is preferably a heavy chain single-domain antibody.

As a preferred example of the present application, a CDR1 amino acid sequence of the VHH1 is as shown in SEQ ID NO: 6; a CDR2 amino acid sequence of the VHH1 is as shown in SEQ ID NO: 8; and a CDR3 amino acid sequence of the VHH1 is as shown in SEQ ID NO: 11.

As another preferred example, the CDR1, CDR2, and CDR3 amino acid sequences of the VHH1 further comprise an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:6, SEQ ID NO:8, and SEQ ID NO:11, respectively; or have one or several amino acid substitutions, deletions, or additions (e.g., 1, 2, 3, 4, or 5 amino acid substitutions, deletions, or additions) compared to the sequences from which they are derived; preferably, the substitution is a conservative substitution.

As a preferred example of the present application, a CDR1 amino acid sequence of the VHH1 is as shown in SEQ ID NO: 7; a CDR2 amino acid sequence of the VHH1 is as shown in SEQ ID NO: 9; and a CDR3 amino acid sequence of the VHH1 is as shown in SEQ ID NO: 12.

As another preferred example, the CDR1, CDR2, and CDR3 amino acid sequences of the VHH1 further comprise an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:7, SEQ ID NO:9, and SEQ ID NO:12, respectively; or have one or several amino acid substitutions, deletions, or additions (e.g., 1, 2, 3, 4, or 5 amino acid substitutions, deletions, or additions) compared to the sequences from which they is derived; preferably, the substitution is a conservative substitution.

As a preferred example of the present application, a CDR1 amino acid sequence of the VHH1 is as shown in SEQ ID NO: 7; a CDR2 amino acid sequence of the VHH1 is as shown in SEQ ID NO: 10; and a CDR3 amino acid sequence of the VHH1 is as shown in SEQ ID NO: 13.

As another preferred example, the CDR1, CDR2, and CDR3 amino acid sequences of the VHH1 further comprise an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:7, SEQ ID NO:10, and SEQ ID NO:13, respectively; or have one or several amino acid substitutions, deletions, or additions (e.g., 1, 2, 3, 4, or 5 amino acid substitutions, deletions, or additions) compared to the sequences from which they is derived; preferably, the substitution is a conservative substitution.

Preferably, the VHH1 comprises or consists of an amino acid sequence as shown in SEQ ID NOs: 1-3.

As another preferred example, the amino acid sequence of the VHH1 further comprises an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NOs: 1-3, respectively; or has one or several amino acid substitutions, deletions, or additions (e.g., 1, 2, 3, 4, or 5 amino acid substitutions, deletions, or additions) compared to the sequences from which it is derived; preferably, the substitution is a conservative substitution.

Preferably, the CDR1, CDR2, and CDR3 amino acid sequences of the VHH2 are as shown in SEQ ID NO:14, SEQ ID NO:15, and SEQ ID NO:17, respectively.

As another preferred example, the CDR1, CDR2, and CDR3 amino acid sequences of the VHH2 further comprise an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:14, SEQ ID NO:15, and SEQ ID NO:17, respectively; or have one or several amino acid substitutions, deletions, or additions (e.g., 1, 2, 3, 4, or 5 amino acid substitutions, deletions, or additions) compared to the sequences from which they are derived; preferably, the substitution is a conservative substitution.

Preferably, the CDR1, CDR2, and CDR3 amino acid sequences of the VHH2 are as shown in SEQ ID NO:14, SEQ ID NO:16, and SEQ ID NO:18, respectively.

As another preferred example, the CDR1, CDR2, and CDR3 amino acid sequences of the VHH2 further comprise an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:14, SEQ ID NO:16, and SEQ ID NO:18, respectively; or have one or several amino acid substitutions, deletions, or additions (e.g., 1, 2, 3, 4, or 5 amino acid substitutions, deletions, or additions) compared to the sequences from which they are derived; preferably, the substitution is a conservative substitution.

Preferably, the VHH2 comprises or consists of an amino acid sequence as shown in SEQ ID NO: 4 or 5.

As another preferred example, the amino acid sequence of the VHH2 further comprise an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:4 or 5, respectively; or has one or several amino acid substitutions, deletions, or additions (e.g., 1, 2, 3, 4, or 5 amino acid substitutions, deletions, or additions) compared to the sequences from which it is derived; preferably, the substitution is a conservative substitution.

Preferably, the above-described transmembrane domain comprises a molecule selected from: one of CD8α, CD28, IgG1, IgG4, 4-1BB, PD-1, CD34, OX40, CD3ε, IL-2 receptor, and IL-7 receptor.

More preferably, the transmembrane domain is selected from CD8α or CD28, and the CD8α transmembrane domain comprises or consists of an amino acid sequence as shown in SEQ ID NO: 19; the CD28 transmembrane domain comprises or consists of an amino acid sequence as shown in SEQ ID NO: 20.

As another preferred example, the amino acid sequence of the transmembrane domain further comprises an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO:19 or 20, respectively; or has one or several amino acid substitutions, deletions, or additions (e.g., 1, 2, 3, 4, or 5 amino acid substitutions, deletions, or additions) compared to the sequences from which it is derived; preferably, the substitution is a conservative substitution.

Preferably, the intracellular signaling domain comprises the following molecules: one or a combination of CD3ζ, FcRγ, FcRβ, CD3γ, CD3δ, CD3ε, TCRζ, CD4, CD5, CD8, CD21, CD22, CD79a, CD79b, CD278, FcεRI, DAP10, DAP12, and CD66d.

More preferably, the above-described intracellular signaling domain is preferably a cytoplasmic signaling sequence of CD3ζ. The cytoplasmic signaling sequence of Cd3ζ has an amino acid sequence as shown in SEQ ID NO: 21.

As another preferred example, the amino acid sequence of the cytoplasmic signaling sequence of Cd3ζ further comprises an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 21; or has one or several amino acid substitutions, deletions, or additions (e.g., 1, 2, 3, 4, or 5 amino acid substitutions, deletions, or additions) compared to the sequences from which it is derived; preferably, the substitution is a conservative substitution.

As another preferred example, the above-described cytoplasmic signaling sequence of Cd3ζ further comprises a wildtype thereof, or a mutant/modified version thereof.

Preferably, the above-described co-stimulatory signal domain comprises a molecule selected from: one or a combination of 4-1BB (CD137), CD27, CD19, CD4, CD28, ICOS (CD278), CD8α, CD82β, BAFFR, HVEM, LIGHT, KIRDS2, SLAMF7, NKp30, NKp46, CD40, CDS, ICAM-1, B7-H3, OX40, DR3, GITR, CD30, TIM1, CD2, CD7, and CD226.

More preferably, the co-stimulatory signal domain is preferably 4-1BB or CD28.The 4-1BB co-stimulatory signal domain comprises or consists of an amino acid sequence as shown in SEQ ID NO: 22; the CD28 co-stimulatory signal domain comprises or consists of an amino acid sequence as shown in SEQ ID NO: 23.

As another preferred example, the amino acid sequence of the co-stimulatory signal domain further comprises an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 22 or 23, respectively; or has one or several amino acid substitutions, deletions, or additions (e.g., 1, 2, 3, 4, or 5 amino acid substitutions, deletions, or additions) compared to the sequences from which it is derived; preferably, the substitution is a conservative substitution.

The above-described chimeric antigen receptor further comprises a hinge region.

Preferably, the hinge region is preferably CD8α. The CD8α hinge region has an amino acid sequence as shown in SEQ ID NO: 24.

As another preferred example, the amino acid sequence of the hinge region further comprises an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 24; or has one or several amino acid substitutions, deletions, or additions (e.g., 1, 2, 3, 4, or 5 amino acid substitutions, deletions, or additions) compared to the sequences from which it is derived; preferably, the substitution is a conservative substitution.

The above-described chimeric antigen receptor further comprises a signal peptide.

Preferably, the signal peptide comprises a molecule selected from: T cell receptor α chain and β chain, CD3ζ, CD3ε, CD4, CD5, CD8, CD9, CD28, CD16, CD22, CD33, CD37, CD45, CD64, CD80, CD86, CD134, CD137, CD154, and GITR, GM-CSF.

More preferably, the signal peptide is preferably CD8α. The CD8α signal peptide has an amino acid sequence as shown in SEQ ID NO: 25.

The above signal peptide, antigen-binding domain, hinge region, transmembrane domain, co-stimulatory signal domain, and signaling sequence are linked in tandem to obtain a particular structure:

L-VHH1-L1-VHH2-F-H-TM-C-CD3ζ (Ia)

where
each "-" is independently a linker peptide or peptide bond;
L is a signal peptide sequence;
VHH1 is the antigen-binding domain that specifically binds to CD38 or CLL1;
VHH2 is the antigen-binding domain that specifically binds to CLL1 or CD38;
L1 is a linker peptide;
F is none or a Flag tag sequence;
H is the hinge region;
TM is the transmembrane domain;
C is the co-stimulatory signal domain; and
CD3ζ is the cytoplasmic signaling sequence derived from CD3ζ, and further comprises a wildtype thereof, or a mutant/modified version thereof.

Preferably, a particular structure that constitutes CAR-T is:

L-VHH1-L1-VHH2- H-TM-C-CD3ζ

each "-" is independently a linker peptide or peptide bond;
L is the CD8a signal peptide molecule;
VHH1 is the antigen-binding domain that specifically binds to CD38 or CLL1;
VHH2 is the antigen-binding domain that specifically binds to CLL1 or CD38;
L1 is a linker peptide;
H is the CD8a hinge region;
TM is the CD8a transmembrane domain;
C is the 4-1BB co-stimulatory domain; and
CD3ζ is the cytoplasmic signaling sequence derived from CD3ζ.

Preferably, a particular structure that constitutes CAR-NK is:

L-VHH1-L1-VHH2-F-H-TM-C-CD3ζ

each "-" is independently a linker peptide or peptide bond;
L is the CD8a signal peptide molecule;
VHH1 is the antigen-binding domain that specifically binds to CD38 or CLL1;
VHH2 is the antigen-binding domain that specifically binds to CLL1 or CD38;
L1 is a linker peptide;
F is a Flag tag sequence;
H is the CD8a hinge region;
TM is the CD28 transmembrane domain;
C is the CD28 co-stimulatory domain; and
CD3ζ is the cytoplasmic signaling sequence derived from CD3ζ.

Preferably, the above linker peptide is selected from linkers of the following amino acid sequences: SGG, GGS, SGGS, SSGGS, GGGG, SGGGG, GGGGS, SGGGGS, GGGGGS, SGGGGGS, SGGGGG, GSGGGGS, GGGGGGGS, SGGGGGGG, SGGGGGGGS, SGGGGSGGGGS, or GGGGSGGGGSGGGGS.

Preferably, the expression tag of the fusion protein is Flag tag, as represented by the amino acid sequence of SEQ ID NO: 27.

As another preferred example, the amino acid sequence of the Flag tag further comprises an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% identity to SEQ ID NO: 27; or has one or several amino acid substitutions, deletions, or additions (e.g., 1, 2, 3, 4, or 5 amino acid substitutions, deletions, or additions) compared to the sequences from which it is derived; preferably, the substitution is a conservative substitution.

A second aspect of the present invention discloses a nucleic acid molecule, the nucleic acid molecule being a nucleotide sequence encoding the above chimeric antigen receptor.

Preferably, the nucleic acid molecule C9B5 encoding the amino acids shown in SEQ ID NO: 2 and SEQ ID NO: 5 is preferred, and the nucleotide sequence of the C9B5 is as shown in SEQ ID NO: 30.

The nucleic acid molecule further comprises a nucleotide sequence as shown in any one of SEQ ID NOs: 28-29 or 31-51.

A third aspect of the present invention discloses a recombinant vector, the vector comprising the chimeric antigen receptor or the nucleic acid molecule.

Preferably, the recombinant vector comprises a DNA vector, an RNA vector, a plasmid, a transposon vector, a CRISPR/Cas9 vector, or a viral vector;
More preferably, the viral vector comprises a lentiviral vector, an adenoviral vector, or a retroviral vector.

A fourth aspect of the present invention discloses an engineered immune cell, the engineered immune cell comprising and/or expressing the chimeric antigen receptor, the nucleic acid molecule, or the recombinant expression vector.

Preferably, the immune cell is prepared using a FAST-CAR process.

More preferably, the FAST-CAR changes activation, transduction, and expansion steps into one single step of "synchronous activation-transduction", in which the engineered immune cell undergoes ex vivo expansion for less than 72 hours.

As another preferred example, the engineered immune cell prepared using the FAST-CAR process can reduce the influence of self-killing caused, during the production process, by CD38 CAR recognizing the CD38 antigen on the surface of the immune cell, or by other CARs recognizing corresponding target antigens on the surface of the immune cell (e.g., CD70 CAR recognizing the CD70 antigen on the surface of a T cell), and the immune cell can be applied to any CAR-targeted antigen and different tumor markers.

As another preferred example, comparing the engineered immune cell prepared using the FAST-CAR process with cells in comparable populations that undergo ex vivo expansion for one week or more weeks, it is observed that the engineered immune cell prepared using the FAST-CAR process is younger in terms of phenotype. The youngness is characterized by higher proportions of T_{n/scm} (CD45RO⁻, CCR7⁺) and Tcm(CD45RO⁺, CCR7⁺).

As another preferred example, comparing the engineered immune cell prepared using the FAST-CAR process with cells in comparable populations that undergo ex vivo expansion for one week or more weeks, it is observed that the engineered immune cell prepared using the FAST-CAR process is stronger in terms of in vitro expansion and persistent killing ability.

Preferably, the immune cell comprises T cell, NK cell, iNKT cell, CTL cell, monocyte, macrophage, dendritic cell, and/or NKT cell.

In another preferred example, the immune cell may preferably be a T cell and an NK cell.

A fifth aspect of the present invention discloses use of the above chimeric antigen receptor, nucleic acid molecule, recombinant expression vector, or engineered immune cell in the preparation of a medicament or pharmaceutical composition for treating a tumor. The tumor may be a cancer. Preferably, the tumor is a tumor or cancer that expresses CD38 and CLL1.

More preferably, the tumor is acute myelogenous leukemia.

Preferably, the pharmaceutical composition further comprises a pharmaceutically acceptable carrier and an adjuvant.

Use of the above chimeric antigen receptor in combination with one or more of the following in the preparation of a pharmaceutical combination for treating and/or preventing a cancer:
(1) an agent for enhancing the efficacy of cells comprising a CAR nucleic acid or a CAR polypeptide;
(2) an agent for improving one or more side effects associated with administration of cells comprising a CAR nucleic acid or a CAR polypeptide; and
(3) an additional agent for treating diseases associated with CD38 and CLL1.

Preferably, the treatment and/or prevention further comprise(s) using a second therapy in combination, the second therapy being selected from surgery, chemotherapy, radiotherapy, immunotherapy, gene therapy, DNA therapy, RNA therapy, nanotherapy, virus therapy, adjuvant therapy, and any combination thereof.

Compared with the prior art, the present invention has the following significant advantages and effects:
(1) The dual-targeting CAR T cell of the present invention exhibits highly specific in vitro cytotoxicity against CLL1-positive and/or CD38-positive target cells.
(2) The dual-targeting CAR T cell of the present invention produced using the FAST process is not influenced by self-killing based on CD38-CAR.
(3) The dual-targeting CAR T cell of the present invention produced using the FAST process is younger in terms of phenotype than a dual-targeting CAR T cell produced using a conventional process, and exhibits more durable tumor-killing ability and expansion ability in in vitro experiments.
(4) The dual-targeting CAR T cell of the present invention produced using the FAST process can effectively inhibit the growth of a CD38 and CLL1-positive tumor in vivo at a lower dose and exhibits a durable anti-tumor efficacy.

### BRIEF DESCRIPTION OF THE DRAWINGS

FIG. 1 is a schematic diagram showing the expression of antigens on the surface of the target cells in Example 1;
FIG. 2 is a schematic structural diagram of the Dual-CAR in Example 2;
FIG. 3 shows the CAR positive rate of Dual-CAR T cells determined using antigens in Example 3;
FIG. 4 is a schematic diagram showing the results of killing of Hela-WT cells and CLL1- and CD38-overexpressing Hela cells by Dual-CAR Ts in Example 4;
FIG. 5 is a schematic diagram showing the results of killing of CLL1- and CD38-expressing target cells by Dual-CAR Ts in Example 4;
FIG. 6 shows a schematic flow chart of flow cytometry analysis of multiple rounds of in vitro killing by CAR-T cells in Example 5;
FIG. 7 is a schematic diagram showing the results of multiple rounds of killing of HL60 cells by Dual-CAR Ts in Example 5;
FIG. 8 is a schematic diagram showing the results of multiple rounds of killing of KG1 cells by Dual-CAR Ts in Example 5;
FIG. 9 is a schematic diagram showing the results of multiple rounds of killing of THP1 cells by Dual-CAR Ts in Example 5;
FIG. 10 is a schematic diagram showing the CAR positive rate after CAR-NK92 sorting in Example 8;
FIG. 11 is a schematic diagram showing the results of killing of tumor cells by CAR-NK92 in Example 9;
FIG. 12 is a schematic diagram showing the results of multiple rounds of killing by CAR-N92K in Example 10;
FIG. 13 is a schematic diagram showing changes in CD38 expression, CAR expression, expansion, and viability of F Dual-CAR Ts after revival in Example 12;
FIG. 14 is a schematic diagram comparing cell differentiation phenotypes of F Dual-CAR T and C Dual-CAR T cells in Example 12;
FIG. 15 is a schematic diagram showing the results of in vitro killing by F Dual-CAR T and C Dual-CAR T cells in Example 13;
FIG. 16 is a schematic diagram showing the results of multiple rounds of in vitro killing by F Dual-CAR T and C Dual-CAR T cells in Example 14; and
FIG. 17 is a diagram showing the in vivo efficacy experiment of F Dual-CAR T and C Dual-CAR T cells in mice in Example 15.

### DETAILED DESCRIPTION OF PREFERRED EMBODIMENTS

The present invention will be further illustrated by specific examples, and it should be understood that the following examples are only for illustrating the present invention, and are not intended to limit the content of the present invention.

The raw materials and equipment used in the examples are well known to those skilled in the art, and are all commercially available or can be readily obtained or prepared.

As used herein, the term "antigen" refers to a molecule or a fragment thereof capable of being bound by a selective binding agent. For example, the antigen may be a ligand that can be bound by a selective binding agent, such as a receptor. As another example, the antigen may be an antigenic molecule that can be bound by a selective binding agent, such as an immune protein (e.g., an antibody). The antigen may also refer to a molecule or a fragment thereof that can be used in an animal to produce an antibody that can bind to the antigen. In some cases, the antigen may bind to a substrate (e.g., a cell membrane). Alternatively, the antigen may not bind to a substrate (e.g., a secreted molecule, such as a secreted polypeptide).

The term "antibody" (Ab) shall include, but is not limited to, an immunoglobulin that specifically binds to an antigen and comprises at least two heavy (H) chains and two light (L) chains interlinked by disulfide bonds, or an antigen-binding moiety thereof. Each H chain comprises a heavy chain variable region (abbreviated herein as VH) and a heavy chain constant region. The heavy chain constant region comprises three constant domains, CH1, CH2, and CH3. Each light chain comprises a light chain variable region (abbreviated herein as VL) and a light chain constant region. The light chain constant region comprises one constant domain, CL. The VH and VL regions may be further subdivided into regions of hypervariability, termed complementarity determining regions (CDRs), interspersed with regions that are more conserved, termed framework regions (FRs). Each of VH and VL comprises three CDRs and four FRs, arranged from an amino terminus to a carboxyl terminus in the following order: FR1, CDR1, FR2, CDR2, FR3, CDR3, FR4. The variable regions of the heavy and light chains contain a binding domain that interacts with an antigen.

It should be understood that the amino acid names herein are identified by internationally accepted single English letters, and the corresponding three-letter abbreviations of the amino acid names are: Ala (A), Arg (R), Asn (N), Asp (D), Cys (C), Gln (Q), Glu (E), Gly (G), His (H), Ile (I), Leu (L), Lys (K), Met (M), Phe (F), Pro (P), Ser (S), Thr (T), Trp (W), Tyr (Y), and Val (V).

The term "complementarity determining region" or "CDR" refers to the hypervariable regions of the heavy and light chains of an immunoglobulin, as defined by Kabat et al. (Kabat et al., Sequences of proteins of immunological interest, 5th Ed", US Department of Health and Human Services, NIH, 1991, and later editions). The present application adopts the Kabat numbering system to define CDRs. There are three heavy chain CDRs and three light chain CDRs. Herein, the terms "CDR" and "CDRs" are used, depending on the context, to refer to a region comprising one or more, or even all, of the major amino acid residues contributing to the binding affinity of an antibody to an antigen or epitope it recognizes. In another specific embodiment, the CDR region or CDR refers to the hypervariable regions of the heavy and light chains of an immunoglobulin as defined by IMGT.

### Preparation of antibody

The antibody of the present invention can be prepared by various methods known in the art, for example, by genetic engineering recombinant technology. For example, DNA molecules encoding the heavy chain and light chain genes of the antibody of the present invention are obtained by chemical synthesis or PCR amplification. The obtained DNA molecule is inserted into an expression vector and then transfected into host cells. Then, the transfected host cells are cultured under specific conditions, whereby the antibody of the present invention is expressed.

The antigen-binding fragments of the present invention can be obtained by hydrolyzing intact antibody molecules (see Morimoto et al., J. Biochem. Biophys. Methods 24: 107-117 (1992) and Brennan et al., Science 229: 81 (1985)). In addition, these antigen-binding fragments can also be directly produced by recombinant host cells (reviewed by Hudson, Curr. Opin. Immunol. 11: 548-557 (1999); Little et al., Immunol. Today, 21: 364-370

(2000)). For example, Fab' fragments can be obtained directly from host cells; Fab' fragments can be chemically coupled to form F(ab')z fragments (Carter et al., Bio/Technology, 10: 163-167 (1992)). In addition, Fv, Fab, or F(ab')z fragments can also be directly isolated from cultures of recombinant host cells. Those skilled in the art are fully aware of other techniques for preparing these antigen-binding fragments.

### Conservative substitution

As used herein, the term "conservative substitution" means an amino acid substitution that does not adversely affect or change the expected properties of a protein/polypeptide comprising an amino acid sequence. For example, conservative substitution can be introduced by standard techniques known in the art, such as site-directed mutagenesis and PCR-mediated mutagenesis. Conservative amino acid substitution includes substitution of an amino acid residue with an amino acid residue having a similar side chain, for example, substitution performed using a residue which is physically or functionally similar to a corresponding amino acid residue (e.g., having similar size, shape, charge, chemical properties, including the ability to form a covalent bond or hydrogen bond, etc.). Families of amino acid residues having similar side chains have been defined in the art. These families include amino acids having a basic side chain (such as lysine, arginine, and histidine), an acidic side chain (such as aspartic acid and glutamic acid), an uncharged polar side chain (such as glycine, asparagine, glutamine, serine, threonine, tyrosine, cysteine, and tryptophan), a nonpolar side chain (such as alanine, valine, leucine, isoleucine, proline, phenylalanine, and methionine), a β-branched side chain (e.g., threonine, valine, isoleucine), and an aromatic side chain (e.g., tyrosine, phenylalanine, tryptophan, histidine). Therefore, it is preferable to substitute the corresponding amino acid residue with another amino acid residue from the same side chain family.

### Chimeric antigen receptor (CAR)

The chimeric antigen receptor (CAR) of the present invention includes an extracellular domain, a transmembrane domain, and an intracellular domain. The extracellular domain includes a target-specific binding element (also referred to as an antigen-binding domain) and a hinge region. The intracellular domain includes a co-stimulatory signaling region and a CD3ζ chain moiety.

The target-specific binding element (also referred to as an antigen-binding domain) refers to an element capable of antigen recognition based on antigen binding specificity.

For the hinge region and the transmembrane region (transmembrane domain), the CAR can be designed to include a transmembrane domain fused to the extracellular domain of the CAR. In some examples, the transmembrane domain can be selected, or modified by amino acid substitutions, to prevent binding of the domain to transmembrane domains of the same or different surface membrane proteins, thereby minimizing interactions with other members of a receptor complex.

A linker may be incorporated between the extracellular domain and the transmembrane domain of the CAR, or between a cytoplasmic domain and the transmembrane domain of the CAR. As used herein, the term "linker" generally refers to any oligopeptide or polypeptide that functions to link a transmembrane domain to an extracellular or cytoplasmic domain of a polypeptide chain. The linker may include 0-300 amino acids, preferably 2-100 amino acids, and most preferably 3-50 amino acids.

The costimulatory signaling region refers to a moiety of the intracellular domain that includes a costimulatory molecule. The costimulatory molecule is a cell surface molecule required for effective response of a lymphocyte to an antigen, rather than an antigen receptor or ligand thereof.

When the CAR of the present invention is expressed in T cells, the antigen-binding domain is fused to the hinge region, the transmembrane region, and the intracellular domain from one or more of the co-stimulatory molecule and the CD3ζ chain.

In another preferred example, the antigen-binding domain is fused to the CD8a hinge region, the CD8a transmembrane region, and the intracellular domain of a combination of the 4-1BB signaling domain and the CD3ζ signal domain.

In another preferred example, the antigen-binding domain is fused to the CD8a hinge region, the CD8a transmembrane region, and the intracellular domain of a combination of the CD28 signaling domain and the CD3ζ signal domain.

### Bispecific CAR targeting CD38 and CLL1

Bispecificity means that the same CAR can specifically bind to and immunologically recognize two different antigens, and the CAR can produce an immune response by binding to any one antigen.

In another preferred example, the bispecific CAR targeting CD38 and CLL1 is as described in the first aspect of the present invention.

In a preferred embodiment of the present invention, the extracellular domain of the CAR provided in the present invention includes an antigen-binding domain targeting CD38 and CLL1.

In another preferred example, the present invention provides a bispecific chimeric antigen receptor against CD38 and CLL1 antigens. The structural components of the CAR that targets both CD38 and CLL1 can include a signal peptide, a single-domain antibody against CD38, a single-domain antibody against CLL1, a hinge region, a transmembrane region, and an intracellular T cell signal region.

The dual-targeting CAR-T has a wider range of treatment. The CAR-immune cell that targets both CD38 and CLL1 can reduce the possibility of antigen escape caused by down-regulation or loss of a single surface antigen. Additionally, the bispecific chimeric antigen receptor against the target combination of CD38 and CLL1 shows highly specific in vitro cytotoxicity.

### Natural killer cells (NK cells)

NK cells are important immune cells in the body, and belong to large granular lymphocytes in terms of morphology. NK cells are derived from bone marrow, and are the third major type of lymphocytes after T cells and B cells, accounting for about 15% of all immune cells (leukocyte count) in the blood. NK cells are the key cells of the natural immune system, and are mainly distributed in the peripheral blood, liver and spleen. In the human body, NK cells are mainly characterized by CD3-CD56+ lymphocyte populations, with the CD16+CD56dim subtype being mainly in the blood (NK cells are classified into two subpopulations of CD56dim and CD56bright according to the difference in the expression density of the CD56 molecule on cells; CD56dim accounts for more than 90% of NK cells, mainly has a cytotoxic effect, expresses the IL-2 receptor (IL-2R) having a medium affinity, and has a stronger killing activity; CD56bright can produce a large number of cytokines, mainly plays an immunomodulatory role, and highly expresses IL-2R).

### Chimeric antigen receptor T cells (CAR-T cells)

As used herein, the terms "CAR-T cells", "CAR-T", and "CAR-T cells of the present invention". Chimeric antigen receptor T cells (CAR-T cells) involve coupling, in vitro, an antigen binding portion of an antibody that can recognize a certain tumor antigen to an intracellular portion of CD3-ζ chain or FcεRIγ to form a chimeric protein, and transfecting T cells of a patient by gene transduction method to express the chimeric antigen receptor (CAR). After the patient's T cells are "re-encoded", a large number of tumor-specific CAR-T cells are generated. The basic principle lies in using the patient's own immune cells to eliminate cancer cells.

CAR-NK adoptive cell therapy (ACT) involves modifying NK cells with a gene of a chimeric antigen receptor (CAR) to impart the NK cells with the ability to target and recognize tumor cells, expanding the NK cells in vitro, and then injecting the NK cells into the human body so as to achieve the effect of tumor treatment. There are currently five main sources of NK cells in clinical use: human peripheral blood (PB), umbilical cord blood (UCB), human embryonic stem cells (hESCs), induced pluripotent stem cells (iPSCs), and NK-92 cell line.

The NK-92 cell line is currently the most widely studied cell line among CAR-NKs, and was isolated from a 50-year-old male with non-Hodgkin lymphoma in 1992. The growth of NK-92 cell line is IL-2-dependent. Compared with primary NK cells, the NK-92 cell line has the greatest advantage that the expression of inhibitory receptors (such as KIR) on its surface is very low. The lack of an inhibitory receptor signal allows the NK-92 cell line to have an ability to kill various tumors better than that of primary NK cells or other killer cells activated by cytokines. In addition, NK-92 also holds a certain potential in the treatment of solid tumors. An important reason for the poor effect of CAR-T in solid tumors is that the tumor cells highly express PD-L1, which binds to the inhibitory molecule PD-1 on the surface of the T cells, thereby inhibiting the killing activity of the T cells. By contrast, the lack of the inhibitory receptor on the surface of NK-92 enables NK-92 to avoid the interference from similar inhibitory signals. However, NK-92 also has some obvious disadvantages, such as potential tumorigenicity and EB virus susceptibility. Therefore, NK-92 must be irradiated before it can be put into use.

### FAST-CAR platform

The "FAST process" as described herein is included as a process for the preparation of engineered immune cells.

In a conventional CAR-T production process, a patient's T cells are first activated using CD3 and/or CD28 antibodies and then transduced with a viral vector to express one or more CARs. These engineered CAR-T cells will be expanded in vitro before being injected back into the human body. Overall, the production process typically takes 1 to 6 weeks.

The FasTCAR platform is capable of simultaneously activating and transducing T cells in resting state by using the XLenti vector derived from lentivirus and having high quality and exhibiting a high gene transduction efficiency. Upon transduction, one or more CARs will be integrated into the T cell genome and be stably expressed. According to our preclinical studies, transduced T cells have high expansion rate and high tumor cell elimination activity, which removes the necessity of an in vitro cell expansion step. The transduced T cells can be directly formulated for administration to patients. Based on such an innovation, the FasTCAR technology can change activation, transduction, and expansion steps into one single step of "synchronous activation-transduction". This technology can significantly shorten the production time of autologous CAR-T cells from the industry standard of 1 to 6 weeks to the completion of production within 72 hours.

### Carrier

A nucleic acid sequence encoding a desired molecule can be obtained using recombinant methods known in the art, such as, for example, by screening cells expressing the gene for a library, by obtaining the gene from a vector known to comprise the gene, or by using a standard technique to directly isolate the gene from cells and tissues comprising the gene. Alternatively, the gene of interest can be produced synthetically.

The present invention further provides a vector into which the expression cassette of the present invention is inserted. Vectors derived from retroviruses such as lentiviruses are suitable tools to achieve long-term gene transfer, because they allow long-term, stable integration of transgenes, and propagation thereof in daughter cells. Lentiviral vectors have an advantage over vectors derived from oncogenic retroviruses such as murine leukemia virus, in that they can transduce non-proliferating cells, such as hepatocytes. They also have the advantage of low immunogenicity.

In brief, the expression cassette or nucleic acid sequence of the present invention is typically operably linked to a promoter and incorporated into an expression vector. The vector is suitable for replication and integration in eukaryotes. Typical cloning vectors include transcriptional and translational terminators, initiation sequences and promoters useful for regulating expression of desired nucleic acid sequences.

The expression construct of the present invention can also be used for nucleic acid immunization and gene therapy using standard gene delivery protocols. Methods for gene delivery are known in the art. See, e.g., U.S. Pat. Nos. 5,399,346, 5,580,859 and 5,589,466, which are incorporated herein by reference in their entireties. In another embodiment, the present invention provides a gene therapy vector.

The nucleic acid can be loaded into many types of vectors. For example, the nucleic acid can be cloned into vectors including, but not limited to, plasmids, phagemids, phage derivatives, animal viruses, and cosmids. Particular vectors of interest include expression vectors, replication vectors, probe generation vectors, and sequencing vectors.

Furthermore, an expression vector can be provided to a cell in the form of a viral vector. Viral vector technology is well known in the art and is described, for example, in Sambrook et al. (2001, Molecular Cloning: A Laboratory Manual, Cold Spring Harbor Laboratory, New York), and other virology and molecular biology manuals. Viruses that can be used as vectors include, but are not limited to retroviruses, adenoviruses, adeno-associated viruses, herpes viruses, and lentiviruses. Typically, suitable vectors include an origin of replication functional in at least one organism, a promoter sequence, a convenient restriction enzyme site, and one or more selectable markers (e.g., WO01/96584; WO01/29058; and US Pat. No. 6,326,193).

A number of virus-based systems have been developed for transferring genes into mammalian cells. For example, retroviruses provide a convenient platform for gene delivery systems. A gene of choice can be inserted into a vector and packaged into retroviral particles using techniques known in the art. The recombinant virus can then be isolated and delivered to a subject cell in vivo or ex vivo. Many retroviral systems are known in the art. In some embodiments, an adenoviral vector is used. Many adenoviral vectors are known in the art. In one embodiment, a lentiviral vector is used.

Additional promoter elements, such as enhancers, can regulate the frequency of transcription initiation. Typically, these are located in a region 30-110 bp upstream of a start site, although it has recently been shown that many promoters further include functional elements downstream of the start site. The spacing between promoter elements is often flexible, so that promoter functions are maintained when the elements are inverted or moved relative to one another. In a thymidine kinase (tk) promoter, the spacing between promoter elements can be increased to 50 bp before the activity begins to decline. Depending on the promoter, it appears that individual elements can function either cooperatively or independently to initiate transcription.

One example of a suitable promoter is the immediate-early cytomegalovirus (CMV) promoter sequence. This promoter sequence is a strong constitutive promoter sequence capable of driving high-level expression of any polynucleotide sequence operably linked thereto. Another example of a suitable promoter is elongation growth factor-1α (EF-1α). However, other constitutive promoter sequences may also be used, including, but not limited to, the simian virus 40 (SV40) early promoter, mouse mammary tumor virus (MMTV), human immunodeficiency virus (HIV) long terminal repeat (LTR) promoter, MoMuLV promoter, avian leukemia virus promoter, Epstein-Barr virus immediate-early promoter, Rous sarcoma virus promoter, and human gene promoters including but not limited to actin promoter, myosin promoter, heme promoter, and creatine kinase promoter. Further, the present invention should not be limited to the use of constitutive promoters. Inducible promoters are also considered part of the present invention. The use of an inducible promoter provides a molecular switch that is capable of turning on expression of a polynucleotide sequence operably linked to the inducible promoter when such expression is desired, or turning off expression when such expression is undesirable. Examples of inducible promoters include, but are not limited to, a metallothionein promoter, a glucocorticoid promoter, a progesterone promoter, and a tetracycline promoter.

In order to evaluate the expression of the CAR polypeptide or a moiety thereof, the expression vector introduced into the cell may further comprise either or both of an optional marker gene or a reporter gene, to facilitate identification and selection of expressing cells from a population of cells sought to be transfected or infected by the viral vector. In other aspects, the optional marker can be carried on a separate fragment of DNA and used in a co-transfection procedure. Both the optional marker and the reporter gene may be flanked by appropriate regulatory sequences, so as to enable expression in host cells. Useful optional markers include, for example, antibiotic resistance genes such as neo, etc.

The reporter gene is used to identify a potentially transfected cell and to evaluate the functionality of a regulatory sequence. Typically, the reporter gene is a gene that is not present in a recipient organism or tissue, or expressed by the recipient organism or tissue, and that encodes a polypeptide whose expression is clearly indicated by some readily detectable properties, such as enzymatic activity. After DNA has been introduced into the recipient cell, expression of the reporter gene is measured at an appropriate time. Suitable reporter genes may include genes encoding luciferase, β-galactosidase, chloramphenicol acetyltransferase, secreted alkaline phosphatase, or green fluorescent protein (e.g., Ui-Tei et al., 2000 FEBS Letters 479:79-82). Suitable expression systems are well known and can be prepared using known techniques or obtained commercially. Typically, a construct having a minimum of five flanking regions that shows the highest level of reporter gene expression is identified as a promoter. Such a promoter region can be linked to a reporter gene and used to evaluate the capability of an agent to modulate promoter-driven transcription.

Methods for introducing genes into cells and expressing genes in cells are known in the art. In the context of an expression vector, the vector can be readily introduced into a host cell, for example, a mammalian, bacterial, yeast, or insect cell, by any method known in the art. For example, the expression vector can be transferred into the host cell by physical, chemical, or biological means.

Physical methods for introducing polynucleotides into host cells include calcium phosphate precipitation, lipofection, particle bombardment, microinjection, electroporation, and the like. Methods for producing cells comprising vectors and/or exogenous nucleic acids are well known in the art. See, e.g., Sambrook et al. (2001, Molecular Cloning: A Laboratory Manual, Cold Spring Harbor Laboratory, New York). A preferred method for introducing polynucleotides into host cells is calcium phosphate transfection.

Biological methods for introducing a polynucleotide of interest into a host cell include the use of DNA and RNA vectors. Viral vectors, particularly retroviral vectors, have become the most widely used method for inserting genes into mammalian, e.g. human, cells. Other viral vectors can be derived from lentiviruses, poxviruses, herpes simplex virus I, adenoviruses, and adeno-associated viruses, among others. See, e.g., U.S. Pat. Nos. 5,350,674 and 5,585,362.

Chemical means of introducing a polynucleotide into a host cell include colloidal dispersion systems, such as macromolecular complexes, nanocapsules, microspheres and beads; and lipid-based systems, including oil-in-water emulsions, micelles, mixed micelles and liposomes. An exemplary colloidal system used as a delivery vehicle in vitro and in vivo is a liposome (e.g., an artificial membrane vesicle).

When a non-viral delivery system is used, an exemplary delivery vehicle is a liposome. The use of lipid preparations is contemplated for introducing nucleic acids into host cells (in vitro, ex vivo, or in vivo). In another aspect, the nucleic acid can be associated with a lipid. Nucleic acids associated with lipids can be encapsulated within the aqueous interior of a liposome, interspersed within the lipid bilayer of the liposome, attached to the liposome by means of a linker molecule associated with both the liposome and an oligonucleotide, entrapped in the liposome, complexed with the liposome, dispersed in a solution comprising a lipid, mixed with the lipid, associated with the lipid, contained in the lipid as a suspension, contained in or complexed with a micelle, or otherwise associated with a lipid. The lipid, lipid/DNA, or lipid/expression vector associated with the composition is not limited to any particular structure in solutions. For example, they may exist in a bilayer structure, such as a micelle, or have a "collapsed" structure. They may also simply be dispersed in solutions, possibly forming aggregates of non-uniform size or shape. The lipids are lipid substances, and may be naturally occurring or synthetic lipids. For example, the lipids include fat droplets that occur naturally in the cytoplasm, as well as compounds comprising long-chain aliphatic hydrocarbons and their derivatives, such as fatty acids, alcohols, amines, amino alcohols, and aldehydes.

### Pharmaceutical composition

The isolated nucleic acid molecule, vector, and host cell of the present invention, as well as the modified immune cell or immune cell composition of the present invention may be prepared into any dosage form known in the medical field, such as tablet, pill, suspension, emulsion, solution, gel, capsule, powder, granule, pulvis, lozenge, suppository, injection (including injection solution, sterile powder for injection and concentrated solution for injection), inhalant, and spray. A preferred dosage form depends on the intended mode of administration and therapeutic use. The pharmaceutical composition of the present invention should be sterile and stable under production and storage conditions. A preferred dosage form is injection. Such an injection may be a sterile injection solution. In addition, the sterile injection solution may be prepared as a sterile freeze-dried powder (for example, by vacuum drying or freeze drying) for convenient storage and use. Such a sterile freeze-dried powder may be dispersed in a suitable carrier before use, such as water for injection (WFI), bacteriostatic water for injection (BWFI), sodium chloride solution (e.g., 0.9% (w/v) NaCl), glucose solution (e.g., 5% glucose), solution comprising a surfactant (e.g., 0.01% polysorbate 20), pH buffer solution (e.g., phosphate buffer solution), Ringer's solution, and any combination thereof.

The isolated nucleic acid molecule, vector, and host cell of the present invention, as well as the modified immune cell or immune cell composition of the present invention may be administered by any suitable method known in the art, including but not limited to oral, buccal, sublingual, eyeball, local, parenteral, rectal, intrathecal, cisternal, inguinal, intravesical, topical (such as powder, ointment or drops), or nasal route. However, for many therapeutic uses, a preferred route/mode of administration may be parenteral administration (for example, intravenous injection or infusion, subcutaneous injection, intraperitoneal injection, and intramuscular injection). Those skilled in the art will understand that the route and/or mode of administration will vary according to the intended purpose. In some embodiments, the isolated nucleic acid molecule, nucleic acid construct, vector, host cell, or the modified immune cell or immune cell composition of the present invention is administered by intravenous injection or infusion.

The pharmaceutical composition of the present invention may include a "therapeutically effective amount" or a "prophylactically effective amount" of the isolated nucleic acid molecule, nucleic acid construct, vector, host cell, or the modified immune cell or immune cell composition of the present invention. The "prophylactically effective amount" refers to an amount sufficient to prevent, stop, or delay the occurrence of diseases. The "therapeutically effective amount" refers to an amount sufficient to cure or at least partially prevent a disease and its complications in a patient who has already had the disease. The therapeutically effective amount of the isolated nucleic acid molecule, nucleic acid construct, vector, host cell, or the modified immune cell or immune cell composition of the present invention may vary depending on the following factors: the severity of the disease to be treated, the overall state of the patient's own immune system, the general situation of the patient such as age, weight, and sex, the mode of drug administration, other treatments administered at the same time, etc.

In the present invention, the administration regimen may be adjusted to obtain the best desired response (such as therapeutic or prophylactical response). For example, the administration may be performed once, or multiple times over a period of time, or the dose may be reduced or increased in proportion to the urgency of the treatment situation.

### Therapeutic use

Therefore, the present invention further provides a method for stimulating a T cell-mediated immune response to a target cell population or tissue of a mammal, including the step of administering the CAR-T cell of the present invention to the mammal.

In one embodiment, the present invention includes a type of cell therapy, which involves separating autologous T cells of a patient (or NK cells of an allogeneic donor source), performing activation and gene modification to produce CAR-T or CAR-NK cells, and then injecting the cells into the body of the patient. In this way, the probability of graft-versus-host disease is extremely low, and an antigen is recognized by the T cells or NK cells in a non-MHC-dependent manner. In addition, a single CAR-T or CAR-NK can treat all cancers that express the antigen. Unlike antibody therapies, the CAR-T or CAR-NK cells can replicate in the body, producing long-term persistence that can lead to sustained tumor control.

In one embodiment, the CAR-T cell of the present invention can undergo robust in vivo T cell expansion and can persist for an extended period of time. Additionally, CAR-mediated immune responses can be part of an adoptive immunotherapy procedure, in which the antigen-binding domain in CAR induces CAR-modified immune cells to produce a specific immune response to the antigen-expressing target cells. For example, anti-CD38 and/or anti-CLL1 CAR-T or CAR-NK cells induce a specific immune response against cells expressing CD38 and/or CLL1.

Although the data disclosed herein specifically disclose a lentiviral vector comprising a CD38 and/or CLL1 antigen recognition and binding domain, hinge and transmembrane regions, a 4-1BB or CD28 co-stimulatory domain, and a CD3ζ intracellular signal domain, the present invention should be construed to include any number of variations to each of the constituent portions of the construct.

The CAR-modified T cell of the present invention can also be used as a type of vaccine for ex vivo immunization and/or in vivo treatment of a mammal. Preferably, the mammal is a human.

For ex vivo immunization, at least one of the following occurs in vitro prior to administering the cell into the mammal: i) cell amplification, ii) introducing a nucleic acid encoding a CAR into the cell, and/or iii) cryopreserving the cell.

Ex vivo procedures are well known in the art and are discussed more fully below. Briefly, the cell is isolated from a mammal (preferably a human) and genetically modified (i.e., transduced or transfected in vitro) with a vector expressing the CAR disclosed herein. The CAR-modified cell can be administered to a mammalian recipient to provide therapeutic benefit. The mammalian recipient can be a human, and the CAR-modified cell can be autologous to the recipient. Alternatively, the cell may be allogeneic, syngeneic, or xenogeneic relative to the recipient.

In addition to the use of a cell-based vaccine for ex vivo immunization, the present invention further provides a composition and a method for in vivo immunization to elicit an immune response against an antigen in a patient.

The present invention provides a method for treating a tumor, comprising administering a therapeutically effective amount of the CAR-modified T cell of the present invention to a subject in need thereof.

The CAR-modified T cell of the present invention can be administered alone or as a pharmaceutical composition with a diluent and/or in combination with other components such as IL-2, IL-17, or other cytokines or cell populations. Briefly, the pharmaceutical composition of the present invention may include a target cell population as described herein, in combination with one or more pharmaceutically or physiologically acceptable carriers, diluents, or excipients. Such a composition may include a buffer such as neutral buffered saline, sulfate buffered saline; a carbohydrates such as glucose, mannose, sucrose, or dextran, mannitol; a protein; a polypeptide or amino acid such as glycine; an antioxidant; a chelating agent such as EDTA or glutathione; an adjuvant (e.g., aluminum hydroxide); and a preservative. The composition of the present invention is preferably formulated for intravenous administration.

The pharmaceutical composition of the present invention may be administered in a manner suitable for a disease to be treated (or prevented). The amount and frequency of administration will be determined by such factors as the patient's condition, and the type and severity of the patient's disease, although appropriate dosages may be determined by clinical trials.

### Example 1: Cell culture and construction

KG1-LucG cells, HL60-LucG cells, U937-LucG cells, THP1-LucG cells, Molm13-LucR cells, Molm13-CLL1-LucR cells, and K562-LucG cells were all cultured using RPMI 1640 medium; 293T and wild-type Hela cells, Hela cells expressing CD38, Hela cells expressing CLL1, and Hela cells expressing both CLL1 and CD38 (Hela-WT, Hela-CD38, Hela-CLL1, and Hela-CLL1-CD38) were cultured using DMEM culture medium. The above culture media were both supplemented with 10% (v/v) fetal bovine serum, 100 U/ml penicillin and streptomycin, 2 mM L-glutamine, and 1 mM sodium pyruvate. The cells were cultured at 37°C and 5% CO₂ under saturated humidity.

The HeLa cells expressing CD38 were a stably transformed cell line obtained by transferring CD38 antigen into Hela cells using a lentiviral vector, and could specifically express CD38 protein molecules; the Hela cells expressing CLL1 were a stably transformed cell line obtained by transferring CLL1 antigen into Hela cells using a lentiviral vector, and could specifically express CLL1 protein molecules; and the Hela cells expressing both CLL1 and CD38 were a stably transformed cell line obtained by further transferring CD38 antigen into Hela cells expressing CLL1 using a lentiviral vector, and could specifically express CLL1 and CD38 protein molecules. The Molm13-LucR cells were a stably transformed cell line obtained by infecting with a lentivirus expressing firefly luciferase-RFP (T2A linking) followed by performing screening. The KG1-LucG cells, HL60-LucG cells, U937-LucG cells, THP1-LucG cells, and K562-LucG cells were stably transformed cell lines obtained by infecting with a lentivirus expressing firefly luciferase-RFP (T2A linking) followed by performing screening. The Molm13-CLL1-LucR cells were a stably transformed cell line obtained by transferring CLL1 antigen into Molm13-LucR cells using a lentiviral vector, and could specifically express CLL1 protein molecules.

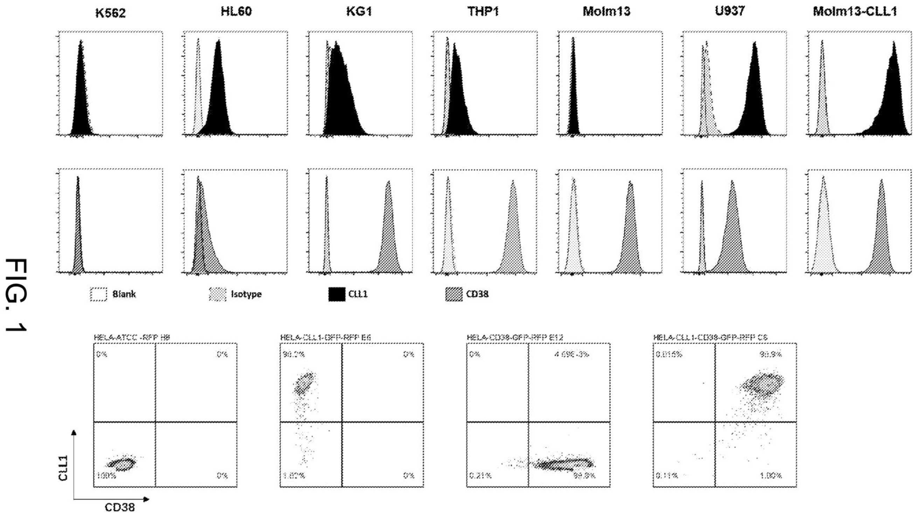

The expression of the antigens on the surface of the target cells used is as shown in Fig. 1. Hela-CD38 represents cells overexpressing CD38, Hela-CLL1 represents cells overexpressing CLL1, Hela-CLL1-CD38 represents cells overexpressing CLL1 and CD38, Hela-WT and K562 represent CLL1 and CD38-double negative cells, Molm13 represents CD38-single positive cells, and HL60, KG1, U937, THP1, and Molm13-CLL1 represent CLL1 and CD38-double positive cells.

### Example 2: Dual CAR vector construction and virus preparation

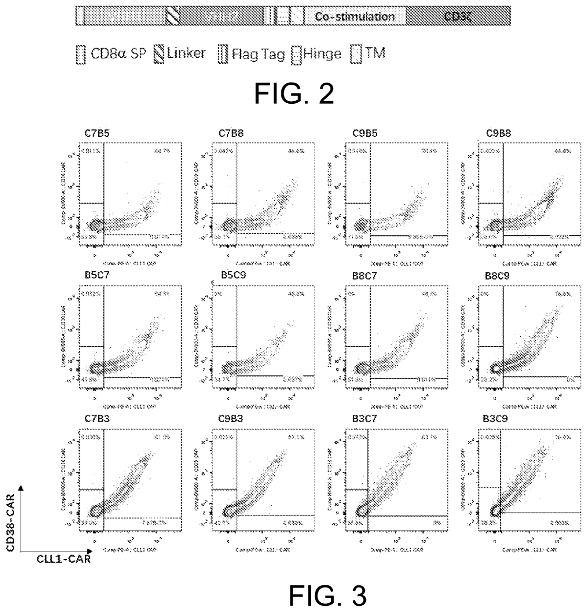

The structure of the CAR targeting both CLL1 and CD38 (Dual CAR) is as shown in FIG. 2, which consisted of CD8α signal peptide, VHH recognizing CD38 (or CLL1), G4S sequence, VHH recognizing CLL1 (or CD38), Flag tag, hinge and transmembrane regions, co-stimulatory signal region, and CD3z signal region. The Dual CAR gene was placed under the promoter of EF1α (EF-1α) to form Dual CAR expression vectors. The Dual CAR expression vectors are designated C7B5, C7B8, C9B5, C9B8, B5C7, B5C9, B8C7, B8C9, C7B3, C9B3, B3C7, and B3C9, respectively, according to the composition of the VHH sequences.

In the designations, B3, B5, B8, C7, and C9 represent single-domain antibodies having the amino acid sequence of B3 alpaca antibody (SEQ ID NO: 1), the amino acid sequence of B5 alpaca antibody (SEQ ID NO: 2), the amino acid sequence of B8 alpaca antibody (SEQ ID NO: 3), the amino acid sequence of C7 alpaca antibody (SEQ ID NO: 4), and the amino acid sequence of C9 alpaca antibody (SEQ ID NO: 5), respectively.

| **No.** | **Description** | **Sequence** |
|---|---|---|
| 1 | Amino acid sequence of B3 alpaca antibody | QVQLVESGGGLVQAGGSLRLACTASGNIFASQVMNWYR QAPGSPRELVATITPGGRTTYADSAKGRFTVATDNAKATA YLQLTSLTPEDTGVYYCHVRLFPATDYWGQGTQVTVSS |
| 2 | Amino acid sequence of B5 alpaca antibody | QVQLVESGGGLVQPGGSLRLSCTASGFRLDYYAIGWFR QAPGKEREAISCIDITGMHTNYGDSVMGRFTISRDDAKR TVYLQMNSLEAEDTGIYYCAPAQYAACSALGVKVSFTSW GQGTQVTVSP |
| 3 | Amino acid sequence of B8 alpaca antibody | QVQLVESGGGLVQPGGSLRLSCTASGFRLDYYAIGWFR QAPGKEREAIACIDITGFHTNYGDSVSGRFTISRDNAGTT VYLQMNSLEAEDTGIYYCAPARYAACSALDVKLSFTSWG QGTQVTVSS |
| 4 | Amino acid sequence of C7 alpaca antibody | QVQLVESGGGLVQPGGSLRLSCAASGFTFSSYAMGWAR QVPGKEVEWVSGIYSDGSTNYADSVKGRFTISRDNAKNT VYLQMNSLRPEDTAVYHCATDPTVHSDLPVWGQGTLVT VSS |
| 5 | Amino acid sequence of C9 alpaca antibody | QVQLVESGGGLVQPGGSLRLSCAASGFTFSSYAMGWAR QVPGKDVEWVSGIYSDGSTYYADSVKGRFTISRYNAKNT VYLQMNSLKPEDTAVYYCATDPTLENQTTAWGQGTQVT VSS |

2.5×10⁶ 293T cells were seeded in DMEM culture medium containing 10% FB in a 150 cm² dish, and the cells were cultured overnight at 37°C and 5% CO₂ under saturated humidity prior to transfection.

On the following day, a helper plasmid and a Dual CAR expression vector were added to a centrifuge tube containing 13.8 mL of OptiMEM culture medium, and then 80 µg of PEI was added to the tube to obtain a mixture. The mixture was left to stand at room temperature for 20 minutes, and then supplemented with 12 mL of OptiMEM culture medium to obtain a transfection culture medium. For transfection, after removing the culture medium, the 293T cells were incubated with the transfection culture medium for 4 to 6 hours, and then the transfection culture medium was replaced with 20 mL of DMEM culture medium containing 2% FBS. After 72 hours, the culture medium was collected and centrifuged at 3000 g and 4°C for 15 min. The supernatant was further centrifuged at 27000 g and 4°C for 2 hours. The pellet was collected and resuspended with 400 µL of pre-cooled X-VIVO culture medium to obtain a Dual CAR lentiviral suspension, which was kept at 4°C overnight. On the following day, the virus suspension was aliquoted for further use.

### Example 3: Preparation of Dual-CAR T cells

All of the T cells were cultured using X-VIVO medium. Pan T cells and CD3/CD28 Dynabeads at a ratio of 1:1 (CD3/CD28 Dynabeads: T cells) and 300 IU/mL of IL2 were incubated together to activate the cells. Two days later, the activated cells were subjected to electrotransfection to knock out the CD38 gene and then subjected to viral transfection to prepare Dual-CAR T.

The specific operation was as follows: 0.25 nmol of gRNA targeting the CD38 gene and 16.5 ug of Cas9 protein were mixed uniformly and incubated at 37°C for 15 min to prepare RNP. During the incubation, 18 uL of supplement buffer was added to 82 uL of Nucleofector Solution to prepare 100 uL of lonza P3 electroporation buffer. At the same time, CD3/CD28 Dynabeads were removed using a magnetic column, and 1 × 10⁷ activated T cells were removed and centrifuged at 400 g for 5 min, and then the cell pellet was collected. After the incubation was completed, the 1 × 10⁷ T cells of the pellet were resuspended using 100 uL of lonza P3 electroporation buffer, and then added to RNP and mixed well. Then the mixture was transferred to an electroporation cup and placed in a lonza 2B electroporator, and electroporation was performed using FI-115 procedure. The electroporated cells were incubated with 300 IU/mL IL2 overnight.

On day 2 after electroporation, the T cells were transfected with the Dual CAR virus at a cell density of 1 × 10⁶ cells/mL at 37°C. The transfected cells were further cultured for expansion. During expansion and cryopreservation, the CAR positive rate of the Dual-CAR T cells was determined using the CLL1 and CD38 antigens. Half of the medium was replaced every 2 to 3 days, and on day 8 after removing CD3/CD28 Dynabeads, the Dual-CAR T cells were harvested.

FIG. 3 shows the CAR positive rate of the Dual-CAR T cells determined using the antigens. The expression of CLL1 CAR and CD38 CAR on the surface of the T cells after viral transfection could be determined using the CLL1 antigen and CD38 antigen at the same time.

### Example 4: In vitro killing by Dual-CAR T cells

An in vitro killing experiment was conducted on the obtained Dual-CAR T cells. RTCA killing analysis was performed using a Hela cell line overexpressing CLL1 and CD38, or determination was performed using luciferase-labeled tumor target cells.

A luciferase gene was introduced into the target cells, and after cloning and screening, stably transformed cell strains (HL60, Molm13, U937, and K562) were obtained. During the experiment, a luciferin substrate was added, and the luciferase reacted with the luciferin to produce fluorescence. The activity of the luciferase could be determined by measuring the intensity of fluorescence, and the killing effect of the CAR-T cells could be obtained by measuring the cell survival rate.

The results showed that after co-culturing the CAR-T cells with the respective target cells (CLL1/CD38 double positive, CLL1 single positive, and CD38 single positive), the target cells were invariably lysed, indicating that the Dual-CAR T invariably had a killing effect on the CLL1/CD38 double positive, CLL1 single positive, and CD38 single positive cells.

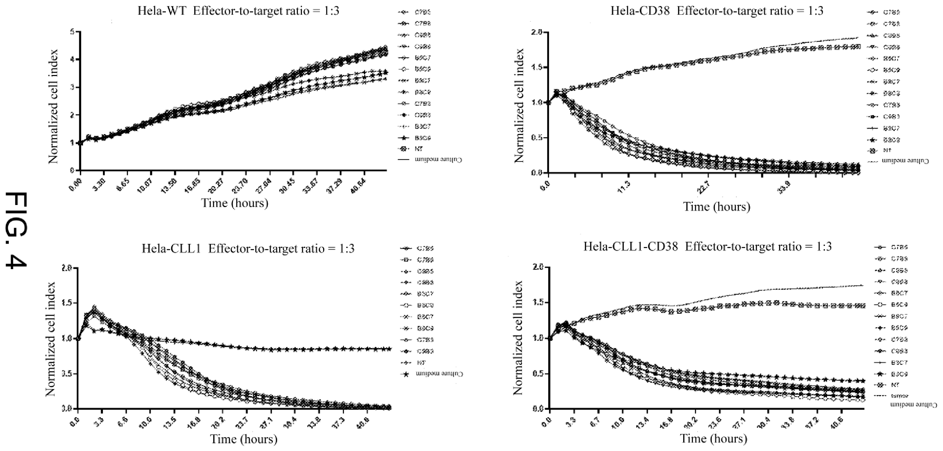

The specific results are as shown in FIG. 4. The bispecific CAR-T had a significant killing effect on the single-positive CLL1-positive target cells (Hela-CLL1) or the single-positive CD38-positive target cells (Hela-CD38), and also had a significant killing effect on the CLL1 and CD38 double-positive target cells Hela-CLL1-CD38. This indicated that the bispecific CAR-T cells having a combination of CLL1 and CD38 had a killing effect on both single-target cells and dual-target cells. In addition, the Dual-CAR T had no killing effect on the Hela target cells that were double negative for CLL1 and CD38.

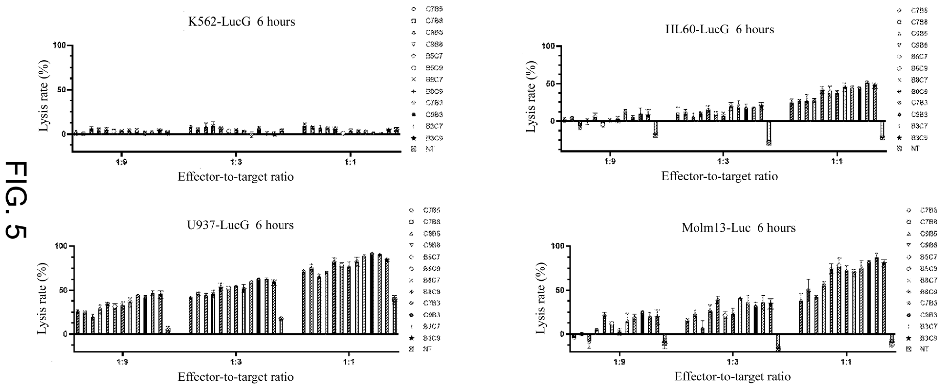

FIG. 5 shows that the Dual-CAR T could significantly kill the HL60, U937, and Molm13 tumor target cells that expressed CD38 and CLL1, and had no killing effect on the K562 cells that were double negative for CLL1 and CD38.

### Example 5: Multiple rounds of in vitro killing by the Dual-CAR T cells

On the first day, the Dual-CAR T cells were revived, and were cultured at a cell density of 1 × 10⁶ cells/mL with 300 IU/mL of IL2 at 37°C. On the second day, samples of 1 × 10⁵ cells were taken, and the proportion of Dual CAR-positive cells was determined using CLL1 and CD38 antigens.

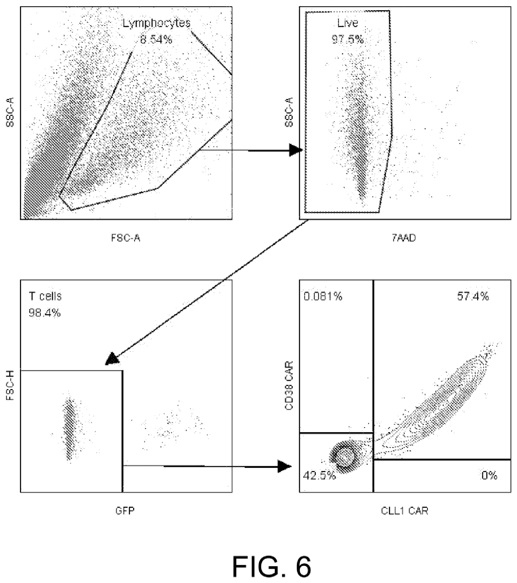

2 × 10⁵ CAR-positive Dual-CAR T cells were taken according to the CAR-positive rate of the cells, and were co-cultured with 6 × 10⁵ HL60-LucG and KG1-LucG cells, respectively (E:T = 1:3) for multiple rounds of killing analysis. On day 3 of each round, part of the co-cultured cells were taken for analysis by flow cytometry. The procedure of the analysis is as shown in FIG. 6, and the specific method is described as follows: A 7AAD-negative viable cell population was circled in a main cell population. Then, T cells (GFP-negative) and target cells (GFP-positive) were distinguished by a GFP signal. In the T cell population, the CAR positive rate was analyzed using CLL1 and CD38 antigen markers. According to the results of flow cytometry and cell counting, the same number of Dual-CAR T cells were taken, and the tumor cells were supplemented to reach a fixed effector-to-target ratio (E:T = 1:3) to perform the next round of killing experiment.

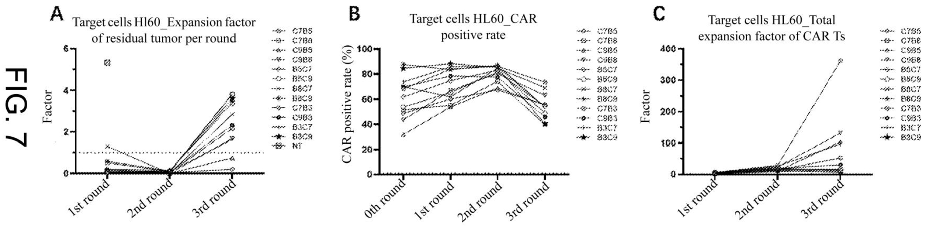

The results of the multiple rounds of killing against the HL60 target cells are as shown in FIG. 7. FIG. 7A shows the expansion factor of the residual cells of the target cells (HL60) at the end of each round for the Dual-CAR T cells; FIG. 7B shows the changes in the CAR positive rate during multiple rounds of killing against the HL60 by the Dual-CAR T cells; and FIG. 7C shows the total expansion factor of CAR T cells during multiple rounds of killing against the HL60 by the Dual-CAR T cells.

It can be observed from FIG. 7A that the inhibitory ability of C7B3 and C9B5 against the HL60 cells was stronger than that of the other Dual-CAR Ts; at the end of the third round, most of the Dual-CAR Ts could no longer inhibit the HL60 cells and the CAR positive rate began to decline (FIG. 7B); and it can be observed from FIG. 7C that during the multiple rounds of killing against the HL60, the expansion of C7B3 was better than that of the other Dual-CAR Ts.

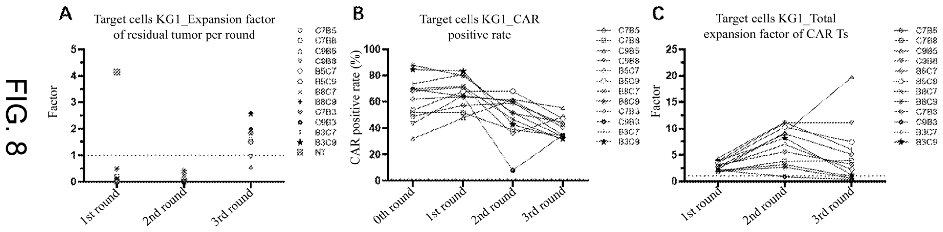

The results of the multiple rounds of killing against the KG1 target cells are as shown in FIG. 8. FIG. 8A shows the expansion factor of the residual cells of the target cells (KG1) at the end of each round for the Dual-CAR T cells; FIG. 8B shows the changes in the CAR positive rate during multiple rounds of killing against the KG1 by the Dual-CAR T cells; FIG. 8C shows the total expansion factor of CAR T cells during multiple rounds of killing against the KG1 by the Dual-CAR T cells.

All the Dual-CAR T cells could not inhibit the KG1 cells well at the end of the third round, but the inhibitory ability of C9B5 and C9B8 against the KG1 cells was stronger than that of the other Dual-CAR Ts (FIG. 8A). It can be observed from FIG. 8C that in the multiple rounds of killing against the KG1, the expansion of C9B5 and C9B8 was better than that of the other Dual-CAR T cells.

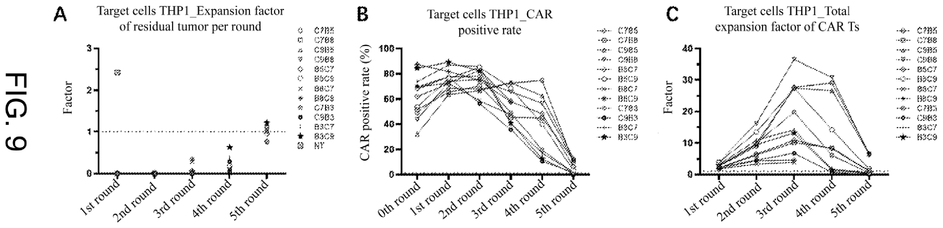

The results of the multiple rounds of killing against the THP1 target cells are as shown in FIG. 9. FIG. 9A shows the expansion factor of the residual cells of the target cells (THP1) at the end of each round for the Dual-CAR T cells; FIG. 9B shows the changes in the CAR positive rate during multiple rounds of killing against the THP1 by the Dual-CAR T cells; FIG. 9C shows the total expansion factor of CAR T cells during multiple rounds of killing against the THP1 by the Dual-CAR T cells.

None of the Dual-CAR T cells could inhibit the THP1 cells well at the end of the fifth round, but the ability of C7B5, C9B5, and C9B8 to sustain CAR expression was stronger than that of the other Dual-CAR Ts (FIG. 9B). It can be observed from FIG. 9C that in the multiple rounds of killing against the THP1, the expansion of C9B8, C7B5, and C9B5 was better than that of the other Dual-CAR T cells.

### Example 6: NK cells and culture method

The complete culture medium for NK92 cells was Alpha MEM supplemented with 12.5% FBS, 12.5% Horse Serum, 1% Pen/strep, 1% sodium pyruvate, 1% L-glutamine, 100U IL2. The complete culture medium for HL60, Molm13, KG-1, THP-1, and K562 cells was RPMI1640 supplemented with 10% FBS, 1% Pen/strep, 1% sodium pyruvate, and 1% L-glutamine. The cells were cultured at 37°C and 5% CO₂ under saturated humidity.

### Example 7: CAR-NK92 lentivirus packaging and transfection

293T cells were revived and seeded into a 150 cm² culture dish, and cultured in a CO₂ incubator for 72 h. After two passages, the cells were seeded into a 150 cm² culture dish for transfection. A four-plasmid system composed of a lentiviral expression vector, helper plasmid gag/pol, Rev, and VSV-G was mixed with PEI transfection reagent, and then added to a certain volume of serum-free DMEM. The mixture was mixed uniformly and left to stand for 15 min. The resulting mixed solution was added to a 150 cm² culture dish on which the 293T cells were spread. The cells were gently mixed uniformly, and cultured in a 5% CO₂ cell incubator at 37°C for 6 h. After 6 h, the culture medium was replaced with fresh culture medium and the culture was continued, and after 48 h and 72 h, the lentiviral culture supernatant was collected for infection. The harvested supernatant was transferred to a centrifuge tube and centrifuged at 4000 rpm for 10 min to remove cell debris. All the centrifuged LVV supernatants were transferred to a 0.45 µm filter for clarification by filtration, and the filtrates were transferred to new centrifuge tubes. The clarified lentiviral supernatants were added to ultracentrifuge tubes and balanced on a balance. The balanced ultracentrifuge tubes were placed in hanging cups, the hanging cup were placed corresponding positions of the rotor, and then placed together in an ultracentrifuge for centrifugation. The centrifugation temperature was 4°C, the rotation speed was 100000 g, the centrifugation time was 90 min, and the acceleration and deceleration were set to the highest. After the ultracentrifugation was completed, the supernatants were discarded, and 0.5 mL of culture medium was added to each tube for resuspension at 2°C to 8°C for 2 h. The harvested virus was added to 1e6 NK92 and mixed in a 24-well plate, and cultured in a CO₂ incubator for 72 h.

### Example 8: CAR-NK flow cytometry detection and sorting

The cell suspension was taken, washed with DPBS three times, and centrifuged at 300 g for 5 min. The supernatant was discarded, an antibody mixed solution (anti-FLAG-APC, 1:100 in DPBS) was added. The mixture was mixed uniformly and placed at 4°C for staining for 30 min. The cells were washed with DPBS three times, and centrifuged at 300 g for 5 min. After resuspension in DPBS, the cells were used for flow cytometry detection and sorting. When performing sorting, PE-positive cells were circled and collected in a 5 ml flow cytometry tube. After the sorting was completed, the cells were centrifuged at 300 g for 5 min, and the supernatant was discarded. The cells were resuspended in a preheated culture medium and placed in a cell culture incubator at 37°C and 5% CO₂.

The sorted CAR-NK92 cells were stained with anti-FLAG-APC antibody, and the expression of CAR was detected by flow cytometry.

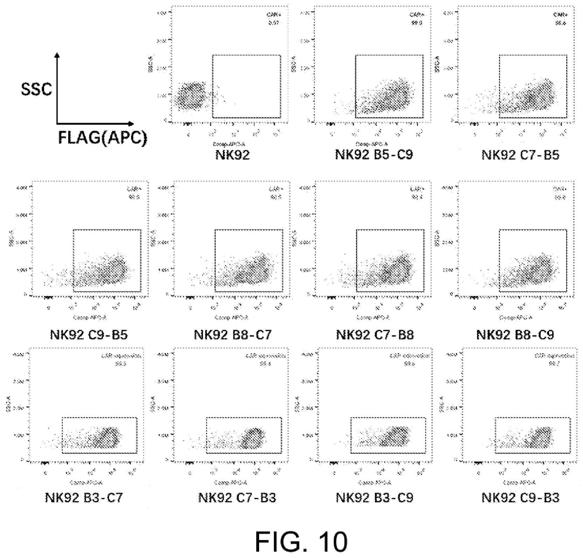

The results of the flow cytometry detection are as shown in FIG. 10. The CAR positive rate of the sorted CAR-NK92 cells all reached 98% or higher.

### Example 9: CAR-NK killing experiment

Luciferase-labeled tumor target cells were used to determine the killing capability. A luciferase gene was transferred into target cells to obtain stably transformed cell lines of Molm13, HL60, THP-1 and KG1 expressing the luciferase gene. During the experiment, a luciferin substrate was added, and the luciferase reacted with the luciferin to produce fluorescence. The activity of the luciferase could be determined by measuring the intensity of fluorescence, and the killing effect of the respective effector cells could be obtained by measuring the cell survival rate.

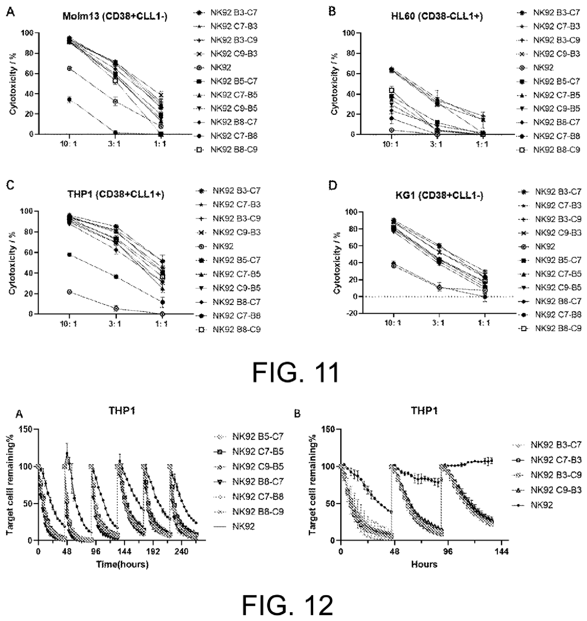

As shown in FIG. 11, all of the CAR-NK92 showed more pronounced killing against the respective target cells compared to NK92, suggesting a CAR-mediated killing effect.

### Example 10: Multiple rounds of CAR-NK killing experiment

On the day before the experiment, the fluorescent target cells THP1 were resuspended and counted. The cell density was adjusted to 8 × 10⁴/ml, and the cells were plated on a 96-well plate at 100 ul per well. On the day of the experiment, corresponding numbers of NK92 and CAR-NK92 cells were added to the wells. The plate was placed in a Cellcyte to start the experiment. Imaging was performed every 4 hours in bright field and fluorescence channels. Every two days were taken as one round. After one round of killing was completed, part of the supernatant was removed, and the cells were transferred to a new plate on which the target cells had been spread. The plate was placed in the Cellcyte to start a new round of killing. Killing Index is calculated as the decrease in the number of fluorescent cells after making adjustments for the zero-crossing moment and for normal cell growth variation.

The results of the multi-round killing experiment are as shown in FIG. 12. The respective CAR-NK92 cells all showed a stronger killing ability than NK92 cells, suggesting that the CAR-mediated killing consistently exerted an effect.

### Example 11: Preparation of Fast Dual-CAR T cells

Peripheral blood was collected from a healthy donor, and PBMCs were isolated by centrifugation at 500 to 600 g for 20 to 30 minutes using a density gradient centrifuge. Magnetic beads that bind CD28 antibodies and CD3 antibodies (CD3/CD28 Dynabeads) were used to sort and enrich for T cells. The T cells bound to CD3/CD28 Dynabeads were further incubated with 300 IU/mL IL2 to activate the cells. At the same time, the cells were transfected, at a cell density of 0.1 to 10×10⁶ cells/mL, with Dual-CAR virus at 37°C overnight. On the next day, the cells were washed with physiological saline buffer and then directly frozen for storage. FAST Dual-CAR T cells could be obtained without further expansion. In this process, the cells were activated, and these cells were also referred to as F Dual-CAR T cells.

### Example 12: Phenotype of Fast Dual-CAR T cells

Due to the presence of the CD38 protein on the surface of the T cells, the expression of CD38 CAR would lead to self-killing, thereby leading to the elimination of the CD38-expressing T cells. In order to analyze whether the Dual-CAR Ts produced by the FAST process would cause severe self-killing, after the Dual-CAR T cells was revived, attention was also paid to the viability, expansion, and cell-surface CD38 expression of the cells while determining the CAR positivity.

The F Dual-CAR T cells were revived and then cultured at a cell density of 1 × 10⁶ cells/mL at 37°C and in the presence of 300 IU/mL of IL2. On each of D2, D3, D5, and D8 after revival, the cells were counted, and a sample of 1 × 10⁵ cells was taken to determine the proportion of the Dual-CAR positive cells using CLL1 and CD38 antigens, and at the same time, the expression of CD38 on the surface of T cells was analyzed using an antibody targeting CD38.

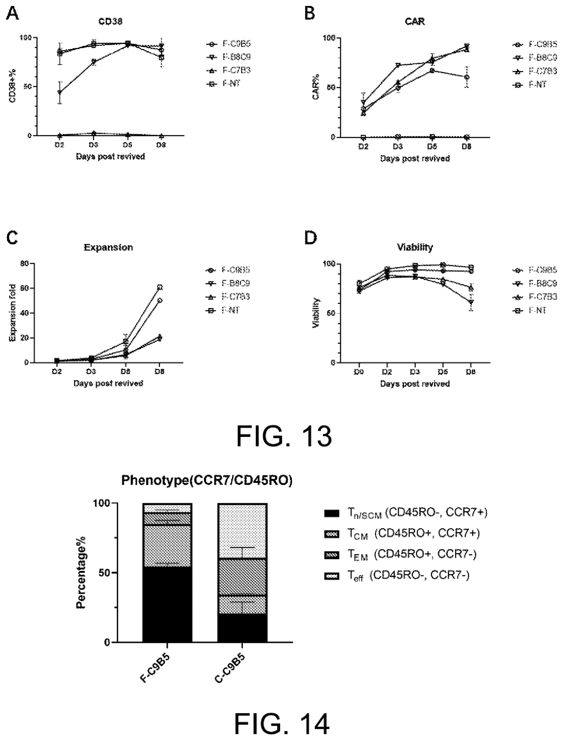

The data of the F Dual-CAR T cells after revival are shown in FIG. 13. FIG. 13A shows the percentage of CD38⁺ on the surface of the T cells. F-C9B5 had a proportion of CD38⁺ similar to that of F-NT; F-B8C9 had a smaller proportion of CD38⁺ cells in the early period of revival, but recovered to a level similar to that of F-NT on the fifth day after revival; in the case of F-C7B3, no CD38⁺ cells could be detected on the cell surface. The CAR positive rate of the respective groups of F Dual-CAR Ts gradually increased after revival (FIG. 13B). F-C9B5 had cell expansion and viability similar to those of F-NT. F-B8C9 and F-C7B3 had lower expansion and viability (FIGS. 13C and B), and it was presumed, in view of the CD38⁺ percentage, that self-killing might have caused the decrease in the cell viability and expansion factor.

On the third day after revival of the F Dual-CAR Ts, the F Dual-CAR Ts and C Dual-CAR Ts (conventional production process) were analyzed for their cell differentiation phenotypes using CD45RO and CCR7. Stem memory-like T cells (Tscm) are defined as CD45RO⁻ CCR7⁺, central memory T cells (Tcm) are defined as CD45RO⁺ CCR7⁺, effector memory T cells (Tem) are defined as CD45RO⁺ CCR7⁻, and terminally differentiated effector T cells (Teff) are defined as CD45RO⁻ CCR7⁻. As shown in FIG. 14, F-C9B5 cells had a greater proportion of young Tscm and Tcm phenotypes, while C-C9B5 cells mostly had the Tem and Teff phenotypes at the later period of differentiation.

### Example 13: In vitro killing experiments of Fast Dual-CAR T cells and C Dual-CAR T cells

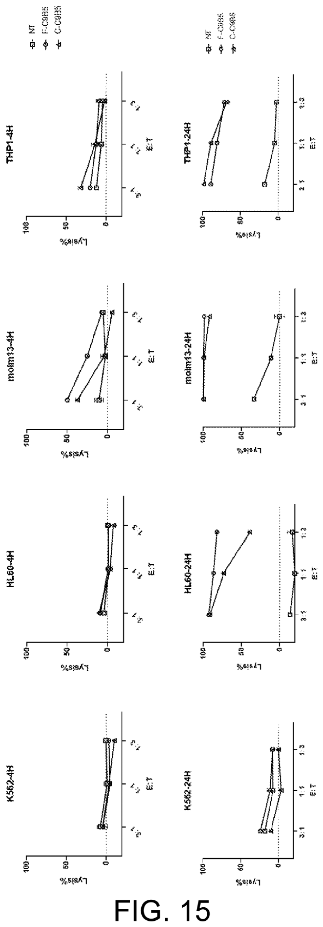

On the first day, the Fast Dual-CAR Ts were revived, and were cultured at a cell density of 1 × 10⁶ cells/mL with 300 IU/mL of IL2 at 37°C. On the second day, the C Dual-CAR T cells were revived, and were cultured at a cell density of 1 × 10⁶ cells/mL with 300 IU/mL of IL2 at 37°C. On the third day, the cells were co-cultured with firefly luciferase-expressing K562, HL60, Molm13, and THP1, respectively, and the killing effect was determined after 4 hours and 24 hours of co-culture, respectively. As shown in FIG. 15, F-C9B5 and C-C9B5 had similar effects in killing target cells.

### Example 14: Multiple rounds of in vitro killing by F Dual-CAR T cells and C Dual-CAR T cells

On the first day, the F CAR Ts were revived, and were cultured at a cell density of 1 × 10⁶ cells/mL with 300 IU/mL of IL2 at 37°C. On the second day, the C CAR Ts were revived, and were cultured at a cell density of 1 × 10⁶ cells/mL with 300 IU/mL of IL2 at 37°C. On the third day, 2 × 10⁵ CAR-positive F Dual-CAR T cells and C Dual-CAR T cells were taken respectively and co-cultured with 1 × 10⁶ HL60, Molm13, and THP1 cells, respectively (E:T being 1:5 in all cases), to perform multiple rounds of killing analysis. On day 3 of each round, 1/6 of the co-cultured cells were taken and directly added to 1 × 10⁶ corresponding target cells to perform the next round of killing experiment. At the same time, part of the co-culture cells were taken and counted, and the proportion of the residual target cells and the proportion of the CAR⁺ cells were analyzed by flow cytometry. The cumulative expansion factor of the CAR T cells could be calculated by taking the results of the counting into consideration.

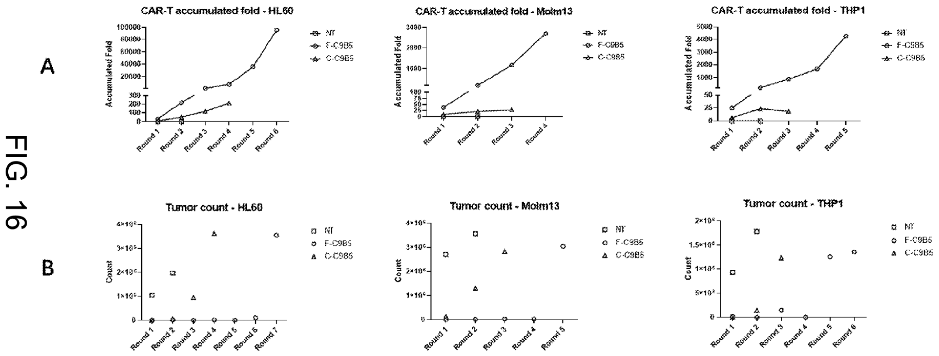

The results of the multiple rounds of killing are as shown in FIG. 16. F-C9B5 could obtain a more durable ability to kill target cells in vitro (FIG. 16B) and a higher expansion factor (FIG. 16A) than C-C9B5, regardless of the target cells.

### Example 15: Comparison of the in vivo efficacy of the F Dual-CAR T cells and C Dual-CAR T cells in mice

Four to six week-old NOG-dKO mice were selected and injected with 2 × 10⁶ Molm13-CLL1-LucR cells via tail vein. Three days later, tumor load was determined through live imaging of small animals. On that day, the mice were grouped and injected with F-C9B5 cells and C-C9B5 cells, respectively. After T-cell treatment, tumor load of the mice was evaluated through live imaging of small animals twice a week.

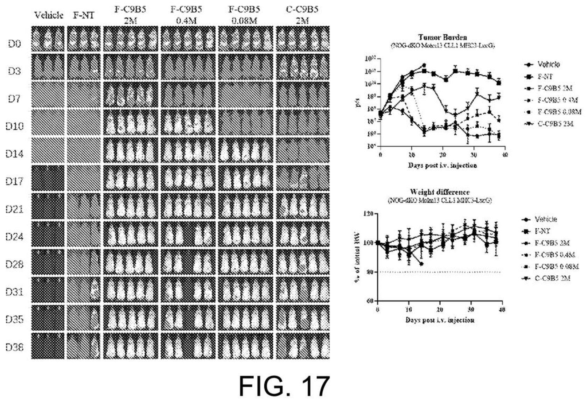

The results are as shown in FIG. 17. Compared with the NT control group, the tumor growth in the mice injected with the CAR T cells was inhibited. The F-C9B5 cells exhibited a dose-related efficacy, and could obtain, at a lower dose, a more durable tumor inhibitory effect than that of C-C9B5. The reinfusion of the respective groups of CAR T cells did not have a significant effect on the body weight of the mice.

So far, those skilled in the art should realize that although several exemplary embodiments of the present invention have been shown and described in detail herein, many other variations or modifications in line with the principles of the present invention can be directly determined or deduced from the disclosure of the present invention without departing from the spirit and scope of the present invention. Therefore, the scope of the present invention should be understood and deemed to cover all such other variations or modifications.

## Claims

1. A bispecific chimeric antigen receptor targeting CD38 and CLL1, a fusion protein of the chimeric antigen receptor at least comprises, from the N-terminus to the C-terminus:
i) an antigen-binding domain that specifically recognizes CLL1 and CD38;
ii) a transmembrane domain;
iii) at least one co-stimulatory domain; and
iv) a signaling domain.

2. The chimeric antigen receptor according to 1, wherein the antigen-binding domain is monovalent or multivalent.

3. The chimeric antigen receptor according to 2, wherein the antigen domain comprises a single-domain antibody.

4. The chimeric antigen receptor according to 3, wherein the single-domain antibody is engineered to be humanized.

5. The chimeric antigen receptor according to 4, wherein the antigen-binding domain comprises two single-domain antibodies VHH1 and VHH2, wherein VHH1 represents a single-domain antibody of a first antigen-binding domain, and VHH2 represents a single-domain antibody of a second antigen-binding domain;
the first antigen-binding domain targets CD38, and the second antigen-binding domain targets CLL1;
the first antigen-binding domain and the second antigen-binding domain are arranged, from the amino terminus to the carboxyl terminus, in one of the patterns selected from the following group:
i) VHH1-VHH2; or
ii) VHH2-VHH1.

6. The chimeric antigen receptor according to 5, wherein a CDR1 amino acid sequence of the VHH1 is as shown in SEQ ID NO: 6; a CDR2 amino acid sequence of the VHH1 is as shown in SEQ ID NO: 8; and a CDR3 amino acid sequence of the VHH1 is as shown in SEQ ID NO: 11.

7. The chimeric antigen receptor according to 5, wherein a CDR1 amino acid sequence of the VHH1 is as shown in SEQ ID NO: 7; a CDR2 amino acid sequence of the VHH1 is as shown in SEQ ID NO: 9; and a CDR3 amino acid sequence of the VHH1 is as shown in SEQ ID NO: 12.

8. The chimeric antigen receptor according to 5, wherein a CDR1 amino acid sequence of the VHH1 is as shown in SEQ ID NO: 7; a CDR2 amino acid sequence of the VHH1 is as shown in SEQ ID NO: 10; and a CDR3 amino acid sequence of the VHH1 is as shown in SEQ ID NO: 13.

9. The chimeric antigen receptor according to any one of claims 6-8, wherein the VHH1 comprises or consists of an amino acid sequence as shown in SEQ ID NOs: 1-3.

10. The chimeric antigen receptor according to 5, wherein a CDR1 amino acid sequence of the VHH2 is as shown in SEQ ID NO: 14; a CDR2 amino acid sequence of the VHH2 is as shown in SEQ ID NO: 15; and a CDR3 amino acid sequence of the VHH2 is as shown in SEQ ID NO: 17.

11. The chimeric antigen receptor according to 5, wherein a CDR1 amino acid sequence of the VHH2 is as shown in SEQ ID NO: 14; a CDR2 amino acid sequence of the VHH2 is as shown in SEQ ID NO: 16; and a CDR3 amino acid sequence of the VHH2 is as shown in SEQ ID NO: 18.

12. The chimeric antigen receptor according to claim 10 or 11, wherein the VHH2 comprises or consists of an amino acid sequence as shown in SEQ ID NO: 4 and SEQ ID NO: 5.

13. The chimeric antigen receptor according to 1, wherein the transmembrane domain is CD8α or CD28.

14. The chimeric antigen receptor according to 13, wherein the CD8α transmembrane domain comprises or consists of an amino acid sequence as shown in SEQ ID NO: 19; and the CD28 transmembrane domain comprises or consists of an amino acid sequence as shown in SEQ ID NO: 20.

15. The chimeric antigen receptor according to 1, wherein the intracellular signaling domain comprises a molecule selected from the following: one or a combination of CD3ζ, FcRγ, FcRβ, CD3γ, CD3δ, CD3ε, TCRζ, CD4, CD5, CD8, CD21, CD22, CD79a, CD79b, CD278, FcεRI, DAP10, DAP12, and CD66d.

16. The chimeric antigen receptor according to 15, wherein the intracellular signaling domain is preferably a CD3ζ cytoplasmic signaling sequence.

17. The chimeric antigen receptor according to 16, wherein the CD3ζ cytoplasmic signaling sequence comprises or consists of an amino acid sequence as shown in SEQ ID NO: 21.

18. The chimeric antigen receptor according to 16, wherein the CD3ζ cytoplasmic signaling sequence further comprises a wildtype thereof, or a mutant/modified version thereof.

19. The chimeric antigen receptor according to 18, wherein the co-stimulatory signal domain comprises a molecule selected from the following: one or a combination of 4-1BB (CD137), CD27, CD19, CD4, CD28, ICOS (CD278), CD82β, BAFFR, HVEM, LIGHT, KIRDS2, SLAMF7, NKp30, NKp46, CD40, ICAM-1, B7-H3, OX40, DR3, GITR, CD30, TIM1, CD2, CD7, and CD226.

20. The chimeric antigen receptor according to 19, wherein the co-stimulatory signal domain is preferably 4-1BB or CD28.

21. The chimeric antigen receptor according to 20, wherein the 4-1BB co-stimulatory signal domain comprises or consists of an amino acid sequence as shown in SEQ ID NO: 22; and the CD28 co-stimulatory signal domain comprises or consists of an amino acid sequence as shown in SEQ ID NO: 23.

22. The chimeric antigen receptor according to 21, wherein the chimeric antigen receptor further comprises a hinge region.

23. The chimeric antigen receptor according to 22, wherein the hinge region is preferably CD8α.

24. The chimeric antigen receptor according to 23, wherein the CD8α hinge region has an amino acid sequence as shown in SEQ ID NO: 24.

25. The chimeric antigen receptor according to 24, wherein the chimeric antigen receptor further comprises a signal peptide;
the signal peptide is selected from the following molecules: one or a combination of T cell receptor α chain and β chain, CD3ζ, CD3ε, CD4, CD5, CD8, CD9, CD28, CD16, CD22, CD33, CD37, CD45, CD64, CD80, CD86, CD134, CD137, CD154, GITR, and GM-CSF.

26. The chimeric antigen receptor according to 25, wherein the signal peptide is preferably CD8α.

27. The chimeric antigen receptor according to 26, wherein the CD8α signal peptide comprises or consists of an amino acid sequence as shown in SEQ ID NO: 25.

28. The chimeric antigen receptor according to 27, wherein the chimeric antigen receptor has the following particular structure:
L-VHH1-L1-VHH2- H-TM-C-CD3ζ
each "-" is independently a linker peptide or peptide bond;
L is the CD8a signal peptide molecule;
VHH1 is the antigen-binding domain that specifically binds to CD38 or CLL1;
VHH2 is the antigen-binding domain that specifically binds to CLL1 or CD38;
L1 is a linker peptide;
H is the CD8a hinge region;
TM is the CD8a transmembrane domain;
C is the 4-1BB co-stimulatory domain; and
CD3ζ is the cytoplasmic signaling sequence derived from CD3ζ.

29. The chimeric antigen receptor according to 27, wherein the chimeric antigen receptor has the following particular structure:
L-VHH1-L1-VHH2-F-H-TM-C-CD3ζ
each "-" is independently a linker peptide or peptide bond;
L is the CD8a signal peptide molecule;
VHH1 is the antigen-binding domain that specifically binds to CD38 or CLL1;
VHH2 is the antigen-binding domain that specifically binds to CLL1 or CD38;
L1 is a linker peptide;
F is a Flag tag sequence;
H is the CD8a hinge region;
TM is the CD28 transmembrane domain;
C is the CD28 co-stimulatory domain; and
CD3ζ is the cytoplasmic signaling sequence derived from CD3ζ.

30. The chimeric antigen receptor according to any one of claims 28-29, wherein the linker peptide is selected from linkers of the following amino acid sequences: SGG, GGS, SGGS, SSGGS, GGGG, SGGGG, GGGGS, SGGGGS, GGGGGS, SGGGGGS, SGGGGG, GSGGGGS, GGGGGGGS, SGGGGGGG, SGGGGGGGS, SGGGGSGGGGS, or GGGGSGGGGSGGGGS.

31. The chimeric antigen receptor according to claim 30, wherein the linker peptide is preferably GGGGSGGGGSGGGGS, represented by the amino acid sequence of SEQ ID NO: 26.

32. The chimeric antigen receptor according to claim 29, wherein the expression tag Flag tag of the fusion protein is represented by the amino acid sequence of SEQ ID NO: 27.

33. A nucleic acid molecule, the nucleic acid molecule being a nucleotide sequence encoding the chimeric antigen receptor according to any one of claims 6-12.

34. The nucleic acid molecule according to claim 33, wherein the nucleic acid molecule is preferably nucleic acid molecule C9B5 encoding the amino acids shown in SEQ ID NO: 2 and SEQ ID NO: 5, and the nucleotide sequence of the C9B5 is as shown in SEQ ID NO: 30.

35. The nucleic acid molecule according to claim 33, wherein the nucleic acid molecule further comprises a nucleotide sequence as shown in any one of SEQ ID NOs: 28-29 or 31-51.

36. A recombinant vector, the recombinant vector comprising the chimeric antigen receptor according to any one of claims 6-12 or the nucleic acid molecule according to any one of claims 33-35.

37. The recombinant vector according to claim 36, wherein the recombinant vector comprises a DNA vector, an RNA vector, a plasmid, a transposon vector, a liposome, a CRISPR/Cas9 vector, or a viral vector.

38. The recombinant vector according to claim 37, wherein the viral vector comprises a lentiviral vector, an adenoviral vector, or a retroviral vector.

39. An engineered immune cell, the engineered immune cell comprising the chimeric antigen receptor according to any one of claims 6-12, or the nucleic acid molecule according to any one of claims 33-35, or the recombinant expression vector according to any one of claims 36-38.

40. The engineered immune cell according to claim 39, wherein the immune cell is prepared using a FAST-CAR process.

41. The engineered immune cell according to claim 40, wherein the FAST-CAR changes activation, transduction, and expansion steps into one single step of "synchronous activation-transduction", in which the engineered immune cell undergoes ex vivo expansion for less than 72 hours.

42. The engineered immune cell according to claim 41, wherein the immune cell can reduce the influence of self-killing caused by CD38 CAR recognizing the CD38 antigen on the surface of the immune cell during the production process, or the influence of self-killing caused by other CARs recognizing corresponding target antigens on the surface of the immune cell during the production process, for example, T cell apoptosis caused by CD70 CAR recognizing the CD70 antigen on the surface of a T cell;
the immune cell can be applied to any CAR-targeted antigen and different tumor markers.

43. The engineered immune cell according to claim 41, wherein the immune cell is younger in terms of cell phenotype compared with cells in comparable populations that undergo ex vivo expansion for one week or more weeks.

44. The engineered immune cell according to claim 43, wherein the youngness is **characterized by** higher proportions of T_{n/scm} (CD45RO-, CCR7+) and Tcm (CD45RO+, CCR7+).

45. The engineered immune cell according to claim 41, wherein compared with cells in comparable populations that undergo ex vivo expansion for one week or more weeks, it is observed that the immune cell is stronger in terms of in vitro expansion and persistent killing ability.

46. The engineered immune cell according to claim 39, wherein the immune cell comprises T cell, NK cell, iNKT cell, CTL cell, monocyte, macrophage, dendritic cell, and/or NKT cell.

47. The engineered immune cell according to claim 46, wherein the immune cell is preferably a T cell and an NK cell.

48. Use of the chimeric antigen receptor according to any one of claims 6-12, the nucleic acid molecule according to any one of claims 33-35, the recombinant expression vector according to any one of claims 36-38, or the engineered immune cell according to any one of claims 39-47 in the preparation of a medicament for treating a tumor.

49. The use according to claim 48, wherein the tumor is a tumor expressing CD38 and/or CLL1.

50. The use according to claim 49, wherein the tumor is acute myelogenous leukemia.

51. The use according to claim 48, wherein the medicament further comprises a pharmaceutically acceptable carrier and an adjuvant.

52. Use of the chimeric antigen receptor according to any one of claims 6-12 in combination with one or more of the following in the preparation of a pharmaceutical combination for treating and/or preventing a cancer:
(1) an agent for enhancing the efficacy of cells comprising a CAR nucleic acid or a CAR polypeptide;
(2) an agent for improving one or more side effects associated with administration of cells comprising a CAR nucleic acid or a CAR polypeptide; and
(3) an additional agent for treating diseases associated with CD38 and CLL1.

53. The use according to claim 52, wherein the treatment and/or prevention further comprise(s) using a second therapy in combination, the second therapy being selected from surgery, chemotherapy, radiotherapy, immunotherapy, gene therapy, DNA therapy, RNA therapy, nanotherapy, virus therapy, adjuvant therapy, and any combination thereof.
